# FASCICULE DE BREVET EUROPEEN

(11) **EP 2 430 163 B1**
(45) Date de publication et mention de la délivrance du brevet: **08.04.2015**
(21) Numéro de dépôt: 10727060.5
(22) Date de dépôt: 28.04.2010
(51) Int. Cl.: C12N 15/62, C07K 1/22

(54) **PROCEDE DE PRODUCTION D'UNE PROTEINE D'INTERET GRACE A UNE PROTEINE DE FUSION**
PROZESS FÜR DIE PRODUKTION EINES BELANGSPROTEINS DURCH EIN FUSIONSPROTEIN
PROCESS FOR THE PRODUCTION OF AN INTERESTING PROTEIN THANKS TO A FUSION PROTEIN.

(30) Priorité: 28.04.2009 FR 0952772
(43) Date de publication de la demande: 21.03.2012
(73) Titulaire: Ecole De Biologie Industrielle (EBI), 95094 Cergy Pontoise Cedex (FR)
(72) Inventeur: EL M'SELMI, Abdellatif, F-75011 Paris (FR)
(74) Mandataire: Cabinet Plasseraud
(86) Numéro de dépôt international: PCT/FR2010/050812
(87) Numéro de publication internationale: WO 2010/125312

(56) Documents cités:
- WO-A1-00/09716
- GIANNONE RICHARD J ET AL: "Dual-tagging system for the affinity purification of mammalian protein complexes" BIOTECHNIQUES, vol. 43, no. 3, septembre 2007 (2007-09), XP002579449 ISSN: 0736-6205
- KOBAYASHI TAKUYA ET AL: "Engineering a Novel Multifunctional Green Fluorescent Protein Tag for a Wide Variety of Protein Research" PLOS ONE, vol. 3, no. 12, décembre 2008 (2008-12), XP002579450 ISSN: 1932-6203
- ARNAU JOS ET AL: "Current strategies for the use of affinity tags and tag removal for the purification of recombinant proteins" PROTEIN EXPRESSION AND PURIFICATION, vol. 48, no. 1, juillet 2006 (2006-07), pages 1-13, XP002579451 ISSN: 1046-5928
- NILSSON J ET AL: "Affinity Fusion Strategies for Detection, Purification, and Immobilization of Recombinant Proteins" PROTEIN EXPRESSION AND PURIFICATION, ACADEMIC PRESS, SAN DIEGO, CA LNKD- DOI:10.1006/PREP.1997.0767, vol. 11, no. 1, 1 octobre 1997 (1997-10-01), pages 1-16, XP004451745 ISSN: 1046-5928

### SEQUENCE LISTING

<110> Ecole de Biologie Industrielle
<120> Procédé de production d'une protéine d'intérêt grâce à une nouvelle protéine de fusion
<130> BCT100127
<160> 29
<170> PatentIn version 3.3
<210> 1
   <211> 486
   <212> DNA
   <213> Spirulina platensis
<400> 1
   atgagtatcg ttaccaaatc catcgtcaat gcagacgctg aagcgcgtta tctgagccct      60
   ggtgaattag atcggatcaa atcctttgtt acctctggcg aacgccgggt tcggattgct     120
   gaaaccatga caggtgctcg tgagcgcatc atcaaggaag caggaaacca acttttccaa     180
   aaacgtcctg atgtagtctc tcccggtgga aatgcctacg gtgaggaaat gactgccacc     240
   tgcctgcggg atctagacta ctacctgcgt ctgatcacct acggaattgt tgctggcgat     300
   gttaccccca ttgaagaaat cggggttgta ggtgttcgcg aaatgtacaa atctcttggt     360
   actcccatcg aagcagtagc tgaaggtgta cgggctatga agagtgtagc cacttccctg     420
   ctgtctggag aagacgcggc cgaagcaggt gcttacttcg actacctaat tggtgcaatg     480
   tcataa                                                                486
<210> 2
   <211> 161
   <212> PRT
   <213> Spirulina platensis
<400> 2
   Met Ser Ile Val Thr Lys Ser Ile Val Asn Ala Asp Ala Glu Ala Arg
   1               5                   10                  15
   Tyr Leu Ser Pro Gly Glu Leu Asp Arg Ile Lys Ser Phe Val Thr Ser
               20                  25                  30
   Gly Glu Arg Arg Val Arg Ile Ala Glu Thr Met Thr Gly Ala Arg Glu
           35                  40                  45
   Arg Ile Ile Lys Glu Ala Gly Asn Gln Leu Phe Gln Lys Arg Pro Asp
       50                  55                  60
   Val Val Ser Pro Gly Gly Asn Ala Tyr Gly Glu Glu Met Thr Ala Thr
   65                  70                  75                  80
   Cys Leu Arg Asp Leu Asp Tyr Tyr Leu Arg Leu Ile Thr Tyr Gly Ile
   85 90 95
   Val Ala Gly Asp Val Thr Pro Ile Glu Glu Ile Gly Val Val Gly Val
   100 105 110
   Arg Glu Met Tyr Lys Ser Leu Gly Thr Pro Ile Glu Ala Val Ala Glu
   115 120 125
   Gly Val Arg Ala Met Lys Ser Val Ala Thr Ser Leu Leu Ser Gly Glu
   130 135 140
   Asp Ala Ala Glu Ala Gly Ala Tyr Phe Asp Tyr Leu Ile Gly Ala Met
   145 150 155 160
   Ser
<210> 3
   <211> 303
   <212> DNA
   <213> Spirulina platensis
<400> 3
   acctctggcg aacgccgggt tcggattgct gaaaccatga caggtgctcg tgagcgcatc 60
   atcaaggaag caggaaacca acttttccaa aaacgtcctg atgtagtctc tcccggtgga 120
   aatgcctacg gtgaggaaat gactgccacc tgcctgcggg atctagacta ctacctgcgt 180
   ctgatcacct acggaattgt tgctggcgat gttaccccca ttgaagaaat cggggttgta 240
   ggtgttcgcg aaatgtacaa atctcttggt actcccatcg aagcagtagc tgaaggtgta 300
   cgg 303
<210> 4
   <211> 101
   <212> PRT
   <213> Spirulina platensis
<400> 4
   Thr Ser Gly Glu Arg Arg Val Arg Ile Ala Glu Thr Met Thr Gly Ala
   1               5                   10                  15
   Arg Glu Arg Ile Ile Lys Glu Ala Gly Asn Gln Leu Phe Gln Lys Arg
               20                  25                  30
   Pro Asp Val Val Ser Pro Gly Gly Asn Ala Tyr Gly Glu Glu Met Thr
           35                  40                  45
   Ala Thr Cys Leu Arg Asp Leu Asp Tyr Tyr Leu Arg Leu Ile Thr Tyr
       50                  55                  60
   Gly Ile Val Ala Gly Asp Val Thr Pro Ile Glu Glu Ile Gly Val Val
   65                  70                  75                  80
   Gly Val Arg Glu Met Tyr Lys Ser Leu Gly Thr Pro Ile Glu Ala Val
                   85                  90                  95
   Ala Glu Gly Val Arg
               100
<210> 5
   <211> 183
   <212> DNA
   <213> Spirulina platensis
<400> 5
   gctgaaacca tgacaggtgc tcgtgagcgc atcatcaagg aagcaggaaa ccaacttttc      60
   caaaaacgtc ctgatgtagt ctctcccggt ggaaatgcct acggtgagga aatgactgcc     120
   acctgcctgc gggatctaga ctactacctg cgtctgatca cctacggaat tgttgctggc     180
   gat                                                                   183
<210> 6
   <211> 61
   <212> PRT
   <213> Spirulina platensis
<400> 6
   Ala Glu Thr Met Thr Gly Ala Arg Glu Arg Ile Ile Lys Glu Ala Gly
   1               5                   10                  15
   Asn Gln Leu Phe Gln Lys Arg Pro Asp Val Val Ser Pro Gly Gly Asn
               20                  25                  30
   Ala Tyr Gly Glu Glu Met Thr Ala Thr Cys Leu Arg Asp Leu Asp Tyr
           35                  40                  45
   Tyr Leu Arg Leu Ile Thr Tyr Gly Ile Val Ala Gly Asp
       50                  55                  60
<210> 7
   <211> 396
   <212> DNA
   <213> Spirulina platensis
<400> 7
   gctgaaacca tgacaggtgc tcgtgagcgc atcatcaagg aagcaggaaa ccaacttttc      60
   caaaaacgtc ctgatgtagt ctctcccggt ggaaatgcct acggtgagga aatgactgcc     120
   acctgcctgc gggatctaga ctactacctg cgtctgatca cctacggaat tgttgctggc     180
   gatgttaccc ccattgaaga aatcggggtt gtagctgaaa ccatgacagg tgctcgtgag     240
   cgcatcatca aggaagcagg aaaccaactt ttccaaaaac gtcctgatgt agtctctccc     300
   ggtggaaatg cctacggtga ggaaatgact gccacctgcc tgcgggatct agactactac     360
   ctgcgtctga tcacctacgg aattgttgct ggcgat                               396
<210> 8
   <211> 132
   <212> PRT
   <213> Spirulina platensis
<400> 8
   Ala Glu Thr Met Thr Gly Ala Arg Glu Arg Ile Ile Lys Glu Ala Gly
   1               5                   10                  15
   Asn Gln Leu Phe Gln Lys Arg Pro Asp Val Val Ser Pro Gly Gly Asn
               20                  25                  30
   Ala Tyr Gly Glu Glu Met Thr Ala Thr Cys Leu Arg Asp Leu Asp Tyr
           35                  40                  45
   Tyr Leu Arg Leu Ile Thr Tyr Gly Ile Val Ala Gly Asp Val Thr Pro
       50                  55                  60
   Ile Glu Glu Ile Gly Val Val Ala Glu Thr Met Thr Gly Ala Arg Glu
   65                  70                  75                  80
   Arg Ile Ile Lys Glu Ala Gly Asn Gln Leu Phe Gln Lys Arg Pro Asp
                   85                  90                  95
   Val Val Ser Pro Gly Gly Asn Ala Tyr Gly Glu Glu Met Thr Ala Thr
               100                 105                 110
   Cys Leu Arg Asp Leu Asp Tyr Tyr Leu Arg Leu Ile Thr Tyr Gly Ile
           115                 120                 125
   Val Ala Gly Asp
       130
<210> 9
   <211> 55
   <212> DNA
   <213> Artificial
<220>
   <223> Primer
<400> 9
   catggactcg tactaccatc accatcacca tcaccatcac catcactgcg gccgc 55
<210> 10
   <211> 51
   <212> DNA
   <213> Artificial
<220>
   <223> primer
<400> 10
   gcggccgcag tgatggtgat ggtgatggtg atggtgatgg tagtacgagg c 51
<210> 11
   <211> 28
   <212> DNA
   <213> Artificial
<220>
   <223> primer
<400> 11
   gcggccgcgg ctcagggatg gacgagaa 28
<210> 12
   <211> 28
   <212> DNA
   <213> Artificial
<220>
   <223> primer
<400> 12
   ttaattaagg taccggatcc tggttcac 28
<210> 13
   <211> 21
   <212> DNA
   <213> Artificial
<220>
   <223> primer
<400> 13
   atcgccgagc actcagacaa g 21
<210> 14
   <211> 18
   <212> DNA
   <213> Artificial
<220>
   <223> primer
<400> 14
   aagggtttcc gaaggctt 18
<210> 15
   <211> 24
   <212> DNA
   <213> Artificial
<220>
   <223> primer
<400> 15
   atcagcagct ttgtggaaaa gatg 24
<210> 16
   <211> 19
   <212> DNA
   <213> Artificial
<220>
   <223> primer
<400> 16
   gctggaactc ctcgccagt 19
<210> 17
   <211> 27
   <212> DNA
   <213> Artificial
<220>
   <223> primer
<400> 17
   atggtacctg attgtcggag gaagtga 27
<210> 18
   <211> 32
   <212> DNA
   <213> Artificial
<220>
   <223> primer
<400> 18
   atgtcgactc aatgtagaaa actttgtatc ca 32
<210> 19
   <211> 29
   <212> DNA
   <213> Artificial
<220>
   <223> primer
<400> 19
   atccatggca attgtcggag gaagtgact 29
<210> 20
   <211> 55
   <212> DNA
   <213> Artificial
<220>
   <223> primer
<400> 20
   atgtcgactc acttttcgaa ctgcgggtgg ctccaatgta gaaaactttg tatcc 55
<210> 21
   <211> 61
   <212> DNA
   <213> Artificial
<220>
   <223> primer
<400> 21
   atccatggct agctggagcc acccgcagtt cgaaaaaggc gccggagaaa gcttgtttaa 60
   g 61
<210> 22
   <211> 20
   <212> DNA
   <213> Artificial
<220>
   <223> primer
<400> 22
   agtacaccat cattcatgag 20
<210> 23
   <211> 26
   <212> DNA
   <213> Artificial
<220>
   <223> primer
<400> 23
   cacgtggtgc cgcgcggcag ccatat 26
<210> 24
   <211> 25
   <212> DNA
   <213> Artificial
<220>
   <223> primer
<400> 24
   cctaggctag gattttttaa tgtta 25
<210> 25
   <211> 769
   <212> DNA
   <213> Artificial
<220>
   <223> 10xHis-SBP-Cyantag®-SalI-site tev-SnabI-site EK-PmlI-NdeI-SwaI-AvrII-xhoI
<400> 25
   ccatggcctc gtactaccat caccatcacc atcaccatca ccatcactgc ggccgcggct 60
   cagggatgga cgagaagacc actggttggc gaggtggaca cgttgttgaa ggactggctg 120
   gggaacttga acaacttcgt gcacgactgg agcatcaccc acaaggtcaa cgtgaaccag 180
   gatccggtac cgatatcagt atcgttacca aatccatcgt caatgcagac gctgaagcgc 240
   gttatctgag ccctggtgaa ttagatcgga tcaaatcctt tgttacctct ggcgaacgcc 300
   gggttcggat tgctgaaacc atgacaggtg ctcgtgagcg catcatcaag gaagcaggaa 360
   accaactttt ccaaaaacgt cctgatgtag tctctcccgg tggaaatgcc tacggtgagg 420
   aaatgactgc cacctgcctg cgggatctag actactacct gcgtctgatc acctacggaa 480
   ttgttgctgg cgatgttacc cccattgaag aaatcggggt tgtaggtgtt cgcgaaatgt 540
   acaaatctct tggtactccc atcgaagcag tagctgaagg tgtacgggct atgaagagtg 600
   tagccacttc cctgctgtct ggagaagacg cggccgaagc aggtgcttac ttcgactacc 660
   taattggtgc aatgtcagag ctcgggcacg tcgacgaaaa cctgtatttt cagggctacg 720
   tagacgacga cgacaagcac gtgcatatga tttaaatcct aggctcgag 769
<210> 26
   <211> 876
   <212> DNA
   <213> Artificial
<220>
   <223> enzyme
<400> 26
   atgcaggatt ctgaatcaaa aaccccaggt gcatcccccc tagtgggcgc agataatgcg 60
   cctttaactg ggggagaagc tgaagggggt gatgcttgga cagtagaaca ggcgatcgcc 120
   gccctaaacc atcaagaacc aaatcgtcgc tattatgcgg cttggtggtt ggggcgtttt 180
   cgtgtgtgtg atgcgatcgc agttgatgcc ttaattatgg ccttggatga tgagtccgat 240
   cgcactaagg acgggggcta tcctttacgc agaaatgcgg ctcgggcttt gggtaaattg 300
   ggcgatcgtc gcgcggtgac tcccctagtc aaatgtttag attgccaaga tttttatgtg 360
   cgggaagcgg cggctcaggc tttagaaggt ctgggagact ctagctgtgt ttctgctttg 420
   cgtgggttat tggtgggtgg agtagaagat actgcaccga tagcgggtaa accccattta 480
   aaacagcctt atgacgcagt aattgaagcc ataggaactc tgggcgcaac tgaggcgtta 540
   actgatattg aacctttcat aaaccatccg atcgctagaa tacagtatgc ggctttaagg 600
   gctatgtatc agcttacggg tgatccggtt tatggcgatc gcttaattgc agctttgcgg 660
   ggggaagatt tacaattgcg acgcagtgct ttaatggatt taggggcgat cggatatctt 720
   caggccgccg aacctatcgc caacacccta gctgaaaata gcctgaaact aattgcttta 780
   cagggaattc tggaacacca tctcagtcaa attggggaaa aatgcctatc tgaagaggca 840
   gttaaaatca tgaagctgat ggattctcta ttatga 876
<210> 27
   <211> 615
   <212> DNA
   <213> Artificial
<220>
   <223> enzyme
<400> 27
   atgacaaagg ttgaggaact aattttagcg gttgaacagg cggactctgc tggaaaactg      60
   ataggcgcgg tgtcacaatt ggccgcgact ggttctgtgg atgcagtacc aatgttaatt     120
   cgtgttttgg gttataacaa ccctggcgcg gcggtggctg cagttgaggg attgatagcc     180
   attggtaaac ctgctgtggt tggtatacta gagaggttag atggttataa ctatggtgct     240
   agggcttggg cggttcgcgc tttgtctggt attggtgatc ctaggggttt agatgttttg     300
   ctggaggcgg cgggtagtga ttttgctctg agtgtgcgtc gtgctgcggc gcggggtttg     360
   ggagcaattc gctgggagga tatgtcacca gaagcagtct ctgaggctaa ggaaagaacc     420
   ctaaaaactc tgctgcatac ttctgaagat acggaatgga tcgttcgtta tgcaacggtt     480
   gtgggtttgg aaggtttggc ggcttctgag gtggataaac cccattggtt tacacaggtt     540
   tcgcaaggtt ggaggggatg gttaataatg atgaaactcc ggcggtttct gctagggcta    600
   gtctggctag gatga                                                      615
<210> 28
   <211> 723
   <212> DNA
   <213> Artificial
<220>
   <223> enzyme
<400> 28
   atgagtgtca acttagcttc ccagttgcgg gaagggacga aaaaatccca ctccatggcg 60
   gagaacgtcg gctttgtcaa atgcttcctc aagggcgttg tcgagaaaaa ttcctaccgt 120
   aagctggttg gcaatctcta ctttgtctac agtgccatgg aagaggaaat ggcaaaattt 180
   aaggaccatc ccatcctcag ccacatttac ttccccgaac tcaaccgcaa acaaagccta 240
   gagcaagacc tgcaattcta ttacggctcc aactggcggc aagaagtgaa aatttctgcc 300
   gctggccaag cctatgtgga ccgagtccgg caagtggccg ctacggcccc tgaattgttg 360
   gtggcccatt cctacacccg ttacctgggg gatctttccg gcggtcaaat tctcaagaaa 420
   attgcccaaa atgccatgaa tctccacgat ggtggcacag ctttctatga atttgccgac 480
   attgatgacg aaaaggcttt taaaaatacc taccgtcaag ctatgaatga tctgcccatt 540
   gaccaagcca ccgccgaacg gattgtggat gaagccaatg acgcctttgc catgaacatg 600
   aaaatgttca acgaacttga aggcaacctg atcaaggcga tcggcattat ggtgttcaac 660
   agcctcaccc gtcgccgcag tcaaggcagc accgaagttg gcctcgccac ctccgaaggc 720
   tag 723
<210> 29
   <211> 747
   <212> DNA
   <213> Artificial
<220>
   <223> enzyme
<400> 29
   atggccgtca ctgatttaag tttgaccaat tcttccctga tgcctacgtt gaacccgatg     60
   attcaacagt tggccctggc gatcgccgct agttggcaaa gtttacccct caagccctat    120
   caattgccgg aggatttggg ctacgtagaa ggccgcctgg aaggggaaaa gttagtgatt    180
   gaaaatcggt gctaccaaac gccccagttt cgcaaaatgc atttggagtt ggccaaggtg    240
   ggcaaagggt tggatattct ccactgtgta atgtttcctg agcctttata cggtctacct    300
   ttgtttggct gtgacattgt ggccggcccc ggtggagtaa gtgcggctat tgcggatcta    360
   tcccccaccc aaagcgatcg ccaattgccc gcagcgtacc aaaaatcatt ggcagagcta    420
   ggccagccag aatttgagca acaacgggaa ttgcccccct ggggagaaat attttctgaa    480
   tattgtttat tcatccgtcc cagcaatgtc actgaagaag aaagatttgt acaaagggta    540
   gtggactttt tgcaaattca ttgtcaccaa tccatcgttg ccgaaccctt gtctgaagct    600
   caaactttgg agcaccgtca ggggcaaatt cattactgcc aacaacaaca gaaaaatgat    660
   aaaacccgtc gggtactgga aaaagctttt ggggaagctt gggcggaacg gtatatgagc    720
   caagtcttat ttgatgttat ccaataa                                        747

## Description

La présente invention concerne un procédé de production d'une protéine d'intérêt grâce à l'utilisation d'une protéine de fusion qui comprend la protéine d'intérêt ainsi qu'un domaine de marquage. En outre, la présente invention concerne une colonne de chromatographie permettant la mise en oeuvre du procédé selon la présente invention.

L'utilisation de protéine d'intérêt, chez l'homme ou l'animal, en particulier en thérapeutique, présente une difficulté majeure liée à son obtention et à sa purification, en ce que le degré de pureté exigé est très élevé et la toxicité de la protéine d'intérêt doit être nulle ou quasi-nulle. Pour obtenir et purifier de telles protéines d'intérêt, il a été proposé dans l'art antérieur d'utiliser des protéines de fusion comprenant ladite protéine d'intérêt associée à un domaine de marquage (ou polypeptide de marquage). De telles méthodes de purification nécessitent une étape de clivage de la protéine de fusion, par une enzyme, permettant ainsi la séparation de la protéine d'intérêt de son domaine de marquage.

Toutefois, ces méthodes de purification de l'art antérieur présentent un rendement faible et un degré de pureté de la protéine d'intérêt faible, ce qui représente un inconvénient majeur pour une production de ces protéines d'intérêt à l'échelle industrielle, comme par exemple dans l'industrie pharmaceutique.

Pour répondre au problème de rendement faible, il a été décrit dans l'art antérieur des méthodes de purification utilisant des enzymes très spécifiques permettant le clivage efficace de la protéine de fusion, aboutissant à l'obtention, d'une part du domaine de marquage, et d'autre part, de la protéine d'intérêt que l'on cherche à produire.

Toutefois, certaines de ces enzymes et le type de clivage qu'elles génèrent, peuvent entrainer de graves problèmes de pureté de la protéine d'intérêt ainsi obtenue. Par exemple, la TEV protéase, largement utilisée, laisse un acide aminé supplémentaire sérine ou glycine ne faisant pas partie de la structure native de la protéine d'intérêt. La présence de cet acide aminé supplémentaire peut provoquer un changement conformationnel de la protéine d'intérêt, une modification de l'activité biologique ou la cristallisation de la protéine d'intérêt et de fait, une réaction immunitaire chez le patient chez qui la protéine d'intérêt est injectée ou administrée.

Pour améliorer le degré de pureté des protéines d'intérêt, WO 00/09716 décrit une méthode de purification basée sur l'utilisation d'une protéine de fusion comprenant deux sites de marquage, ce qui permet de réaliser au moins deux étapes successives de purification et donc d'obtenir une purification améliorée.

R.J. Giannone et al.,Biotechniques 43:296-302, september 2007 décrit aussi un système de dual-Tagging avec différentes combinaisons de marqueurs pour la purification par affinité de complexes de protéines de mammifères.

Toutefois, une telle méthode n'apporte pas en fait une solution acceptable pour répondre au problème de degré de pureté de la protéine d'intérêt car le clivage de la protéine de fusion, entre le domaine de marquage et la protéine d'intérêt, se fait par ajout de l'enzyme dans le milieu contenant la protéine de fusion. Cet ajout conduit, certes à la libération de la protéine d'intérêt, mais également à la contamination, par l'enzyme, du milieu contenant la protéine d'intérêt purifiée.

Ainsi, les méthodes de purification de protéine d'intérêt dans l'art antérieur, conduisent à l'obtention de protéines d'intérêt contaminées par des enzymes et/ou par des polypeptides de marquage, ce qui se traduit par un degré de pureté faible des protéines d'intérêt ainsi obtenues.

La présence de polypeptide de marquage et/ou d'enzyme de clivage conduit également à une toxicité importante des protéines d'intérêt, en particulier dans le cadre d'une utilisation des protéines d'intérêt à des fins thérapeutiques, alimentaires ou cosmétiques.

En outre, d'autres méthodes de production, décrites dans l'art antérieur, utilisent comme marqueur dans le domaine de marquage de la protéine de fusion des composés auto-fluorescents tel que la GFP, dont l'utilisation nécessite une excitation ultra violette qui peut s'avérer toxique pour la cellule recombinante. En outre, les travaux de Liu and al, *Is green fluorescent protein toxic* to *the living cells* ?, publié en 1999 ont montré que l'expression de la GFP pouvait induire l'apoptose de la cellule recombinante, ce qui empêche la mise en place de lignées cellulaires stables exprimant la GFP, et donc exprimant une protéine de fusion ayant un domaine de marquage comprenant la GFP.

Ainsi, l'utilisation de la GFP peut avoir des conséquences néfastes sur une cellule recombinante utilisée pour produire une protéine de fusion comprenant une protéine d'intérêt, et donc sur le rendement final et la qualité de la protéine d'intérêt.

Enfin, aucun document ne décrit dans l'art antérieur de procédés permettant le suivi de la production et la quantification d'une protéine de fusion de manière efficace et sans toxicité pour la cellule recombinante. En particulier, il n'est pas décrit dans l'art antérieur de procédés de suivi de la production et de la quantification en temps réel, c'est-à-dire au cours de la production. En effet, aucune technique actuellement utilisée ne permet la vérification de la production de la protéine de fusion par la cellule recombinante avant la fin de la culture cellulaire.

Ainsi, il existe aujourd'hui un besoin, notamment dans l'industrie pharmaceutique en recherche, notamment pour la production et la purification de cibles thérapeutiques, pour la recherche de candidats médicaments et dans le domaine de protéines thérapeutiques, pour un procédé de production de protéine d'intérêt grâce à l'utilisation d'une protéine de fusion, procédé qui permet d'obtenir une protéine d'intérêt présentant un degré de pureté maximum, dans un rendement important et surtout qui permet d'obtenir une protéine d'intérêt ayant une toxicité quasi nulle voire nulle.

La présente invention répond à ce besoin en proposant un procédé de purification simple permettant l'obtention d'une protéine d'intérêt avec un rendement et un degré de pureté important et une toxicité minimale, voire nulle. Ce procédé s'appuie sur l'utilisation d'une protéine de fusion qui comprend une protéine d'intérêt ainsi qu'un domaine de marquage constitué d'une séquence d'histidine, d'un domaine d'interaction à la streptavidine et d'un marqueur coloré, le domaine de marquage et la protéine d'intérêt étant séparés par un site de clivage spécifique de l'entérokinase ou la TEV protéase.

En outre, le procédé de la présente invention permet le suivi et la quantification de la production, notamment en temps réel, ce qui permet d'une part de vérifier la production de la protéine de fusion, et d'autre part, de moduler le milieu réactionnel pour optimiser le procédé de production de la protéine de fusion et ainsi améliorer le rendement. Un tel procédé permet d'accélérer la mise au point des procédés de production et améliore ainsi sensiblement le rendement de la production de la protéine de fusion.

Enfin le procédé de l'invention prévoit l'utilisation d'une colonne de chromatographie sur laquelle est fixée au moins une enzyme spécifique du site de clivage enzymatique présent sur la protéine de fusion, ledit site de clivage séparant la protéine d'intérêt du domaine de marquage. Une telle colonne présente un avantage majeur car elle peut être réutilisée et permet ainsi l'amortissement économique du prix onéreux des enzymes de clivage. Enfin, la fixation d'au moins une enzyme sur la colonne de chromatographie permet d'une part la récupération de la protéine d'intérêt purifiée sans étape supplémentaire de séparation de la protéine d'intérêt de l'enzyme ni de partenaires de fusions, ce qui simplifie sensiblement la purification, et d'autre part l'absence de contamination par des enzymes de clivage et des domaines de marquages du milieu contenant la protéine d'intérêt.

Ainsi, la présente invention concerne une protéine de fusion caractérisée en ce qu'elle comprend deux domaines distincts :
✔ un domaine de marquage, comprenant les éléments suivants :
   ➢ une séquence d'histidines, comprenant au moins 6 histidines, préférentiellement 10 histidines ;
   ➢ un domaine d'interaction avec la streptavidine, préférentiellement la streptavidin binding protein (SBP),
   ➢ un marqueur, de préférence coloré et plus préférentiellement choisi dans le groupe comprenant la Spirulina platensis allophycocyanin alpha subunit et le cytochrome b5 ainsi que leurs mutants ;
   et
✔ une protéine d'intérêt,
le domaine de marquage et la protéine d'intérêt étant séparés par une séquence de clivage spécifique de l'entérokinase ou de la TEV protéase.

Préférentiellement, l'invention concerne une protéine de fusion caractérisée en ce qu'elle comprend de sa partie amino terminale à sa partie carboxy terminale deux domaines distincts :
✔ un domaine de marquage, comprenant les éléments suivants :
   ➢ une séquence d'histidines, comprenant au moins 6 histidines, préférentiellement 10 histidines ;
   ➢ un domaine d'interaction avec la streptavidine, préférentiellement la streptavidin binding protein,
   ➢ un marqueur, de préférence coloré et choisi dans le groupe comprenant comprenant la Spirulina platensis allophycocyanin alpha subunit et le cytochrome b5 ainsi que leurs mutants ;
   et
✔ une protéine d'intérêt,
ce domaine de marquage et cette protéine étant séparés par une séquence de clivage spécifique de l'entérokinase ou la TEV protéase.

De manière préférée, la protéine de fusion selon l'invention est caractérisée en ce que la séquence de clivage séparant le domaine de marquage de la protéine d'intérêt est spécifique de la TEV protéase si le marqueur coloré est la Spirulina platensis allophycocyanin alpha subunit.

De manière plus préférée, la protéine de fusion selon l'invention est caractérisée en ce que la séquence de clivage séparant le domaine de marquage de la protéine d'intérêt est spécifique de l'entérokinase.

De manière encore plus préférée, la protéine de fusion selon l'invention est caractérisée en ce que la séquence de clivage séparant le domaine de marquage de la protéine d'intérêt est spécifique de l'entérokinase et le marqueur coloré est la Spirulina platensis allophycocyanin alpha subunit.

Par **"protéine de fusion"**, on entend une construction qui renferme plusieurs protéines ou polypeptides d'origine différente. Cette protéine de fusion est codée par un acide nucléique obtenu par les technologies d'ADN recombinant, bien connues de l'homme du métier.
Dans la plupart des cas, une de ces protéines est celle que l'on veut étudier tandis que l'autre lui confère des propriétés qui la rendent facile à détecter et à purifier. Dans le cadre de cette invention, la protéine de fusion est constituée d'un polypeptide, qui est un domaine de marquage, et d'une protéine d'intérêt.

Par **clonage**, on entend l'amplification de fragments d'intérêts par PCR, digestion enzymatique (vecteurs et inserts), ligation, transformation, criblage et validation des séquences par séquençage.

Par **"polypeptide"**, on entend un peptide, un oligopeptide, un oligomère ou une protéine comprenant au moins deux acides aminés joints l'un à l'autre par une liaison peptidique normale ou modifiée.
Un polypeptide peut être composé d'acides aminés autres que les 20 acides aminés codés par les gènes humains. Un polypeptide peut également être composé d'acides aminés modifiés par des processus naturels, tels que le processus de maturation post-traductionnelle ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.
Dans le cadre de cette invention, les polypeptides peuvent contenir jusqu'à 4000 acides aminés. On réservera le terme de protéine à un polypeptide qui possède une structure tridimensionnelle bien définie.

Par **"domaine"**, on entend un fragment polypeptidique, qui se distingue d'un autre fragment par sa nature ou ses fonctions.

Par **"domaine d'interaction avec la streptavidine"**, on entend un fragment de polypeptide, un polypeptide ou une protéine présentant une affinité, c'est-à-dire une attraction chimique, pour la streptavidine, qui est une protéine tétramérique de 60kD, extracellulaire produite par *Streptomyces avidinii* et ayant quatre sites de liaison à la biotine. La streptavidine lie la biotine avec une grande affinité (K_{d}= 10⁻¹⁴). Ces sites de liaisons à la biotine permettent l'association streptavidine-biotine-peroxydase.

Dans le cadre de l'invention, on peut utiliser comme domaine d'interaction avec la streptavidine, la « Streptavidin binding protein » (SBP) ou le streptag II. L'utilisation de la SBP dans le domaine de marquage de la protéine de fusion selon l'invention a pour conséquence la fixation dudit polypeptide sur une colonne recouverte de streptavidine.

Par **"séquence d'histidines"**, on entend un enchainement polypeptidique d'acides aminés, principalement d'histidines, et pouvant présenter d'autres acides aminés. Selon la présente invention, la séquence d'histidines présente préférentiellement 10 histidines consécutives. Alternativement, la séquence d'histidines présente 6 histidines consécutives.

Par **"marquage"** on entend l'opération consistant à utiliser des marqueurs pour la détection et/ou la quantification du polypeptide objet de l'invention.

Par **"marqueur coloré"**, on entend toute substance colorée, naturelle, artificielle ou synthétique, absorbant la lumière dans le domaine de l'ultra-violet, le visible et/ou l'infrarouge. Dans le cadre de l'invention on utilisera préférentiellement la phycocyanine et ses mutants le cytochrome b5 et ses mutants, et la phycoérythrine.
Le cytochrome b5, de couleur rouge, possède un maximum d'absorption à 412 nm.

Par **"phycocyanine"**, on entend la Spirulina platensis allophycocyanin alpha subunit, dont le nom a été déposé sous l'appellation Cyantag®. La phycocyanine est issue de la spiruline et est un pigment naturel de couleur bleue. Elle possède un maximum d'absorption à 626 nm et émet à 640 nm. La séquence SEQ ID N°2 correspond au polypeptide de la Spirulina platensis allophycocyanin alpha subunit, codé par la séquence polynucléotique SEQ ID N°1.

Par **"mutant"**, on entend un polypeptide présentant une séquence modifiée et obtenue selon des techniques de mutagénèse bien connue de l'homme du métier. Ces mutants sont obtenus par mutagenèse dirigée. La mutagenèse dirigée est une technique visant à modifier artificiellement la séquence nucléotidique d'un fragment d'acide nucléique, dans le but de modifier l'activité biologique du polypeptide pour lequel ledit fragment code.
Dans le cadre de cette invention, l'utilisation de mutants du cytochrome b5 et de la phycocyanine a pour intérêt majeur une amélioration de la coloration par rapport au cytochrome b5 sauvage.
Da manière préférée, les mutants de la phycocyanine dans le cadre de la présente invention sont les polypeptides SEQ ID N° 4, 6 ou 8, respectivement codés par les séquences polynucléotidiques SEQ ID N° 3, 5 ou 7.

Par **"protéine d'intérêt"**, on entend une protéine, un fragment d'une protéine ou un peptide qui présente un intérêt, par exemple biologique (dans la recherche), biotechnologique, thérapeutique ou vaccinal, pour l'homme ou l'animal.
Cette protéine peut-être par exemple MyRIP (myosine-VIIa-and Rab- interacting protein) l'erythropoïétine, l'insuline, les interférons, les cytokines (oncostatin M humaine ou encore l'hormone de croissance.

Par **"séquence de clivage spécifique"**, on entend une séquence polypeptidique spécifiquement reconnue par une enzyme. La présence de ce site de clivage permet de séparer, grâce à l'action de l'enzyme sur cette séquence de clivage spécifique, le polypeptide en au moins 2 fragments.

Dans le cadre de la présente invention, les enzymes utilisées sont l'entérokinase et la TEV protéase ou encore une protéase de fusion telle que l'entérokinase-streptagII-TEV protéase ou l'entérokinase-SBP-TEV.
Ces protéases de fusion offrent aux laboratoires de recherches d'utiliser la même colonne pour deux sites différents, à savoir la TEV protéase et l'entérokinase.

La chaine légère de l'entérokinase, que l'on appelle également l'enteropeptidase est une serine protéase, de masse moléculaire de 26 kDa (forme tronquée de l'entérokinase à 150KD) qui reconnait la séquence suivante :
-Asp-Asp-Asp-Asp-Lys-X
Où X est quelconque acide amine et où la coupure se fait entre la lysine, précédée de 4 acides aspartique, et le X.

La Tev protéase (27 KDa) issue du virus du tabac, reconnaît un site spécifique de 7 acides aminés (E-X-X-Y-X-Q-S) et coupe entre la glutamine et la sérine. Le X correspond à plusieurs acides aminés possibles, toutefois. La séquence optimale de cette enzyme (E-N-L-Y-F-Q-S) est très rare ce qui explique sa spécificité.

Il peut y avoir plusieurs séquences de clivage sur un même polypeptide. Par exemple, s'il existe 2 séquences de clivage sur un polypeptide, ledit polypeptide sera clivé en 3 morceaux.

Par **"position carboxy terminale"**, on entend la position la plus proche du groupement carboxylique libre d'un polypeptide.

Par **"position amino-terminale"**, on entend la position la plus proche du groupement amine libre d'un polypeptide.

L'invention concerne également un acide nucléique caractérisé en ce qu'il code pour la protéine de fusion selon l'invention.

Par **"acide nucléique"**, on entend toute séquence ou chaîne nucléotidique simple brin ou double brin pouvant être de type ADN ou ARN, préférentiellement de l'ADN. Le terme acide nucléique englobe toutes les molécules d'acides nucléiques qui se retrouvent à l'état naturel ou artificiel. Ceci inclut les molécules d'ADN, les molécules d'ARN, les ADNc, les séquences exprimées (ESTs), les séquences artificielles et tous les fragments de ceux-ci. On entend également par acide nucléique, les ADN et ARN contenant une ou plusieurs bases modifiées. On entend par base modifiée, par exemple, les bases inhabituelles telles que l'Inosine. Le terme acide nucléique vise également les acides nucléiques de forme modifiée chimiquement, enzymatiquement ou métaboliquement. Typiquement, les acides nucléiques de la présente invention peuvent être préparés par les techniques classiques de biologie moléculaire ou par synthèse chimique.

L'invention concerne également une cassette d'expression comprenant l'acide nucléique codant pour la protéine de fusion selon l'invention.

Par **"cassette d'expression"**, on entend une construction susceptible d'être inséré sur un vecteur et qui comporte tous les éléments nécessaires à l'expression de la protéine de fusion selon l'invention, en utilisant les procédés tels que bien connus par l'homme du métier. Plus spécifiquement, la cassette d'expression selon l'invention comprend un acide nucléique ainsi qu'une séquence régulatrice ; cet acide nucléique étant préférentiellement de l'ADN, qu'on appellera par la suite la séquence codante.
Concernant les cassettes d'expression selon l'invention, tout type de séquence promotrice peut être utilisé. Le choix du promoteur dépendra notamment de l'organisme hôte choisi pour l'expression du gène d'intérêt. Parmi les promoteurs fonctionnels dans les bactéries, on citera notamment celui de la RNA polymérase du bacteriophage T7 (Studier et al., Methods in enzymology 185:60-89, 1990). Ces promoteurs sont décrits dans la littérature et bien connus de l'homme du métier.
Les cassettes d'expression peuvent en outre inclure toute autre séquence nécessaire à l'expression des polypeptides, comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer").
En outre, les cassettes d'expression peuvent inclure toute autre séquence nécessaire à la sécrétion des polypeptides produits par l'organisme hôte telles que des séquences signal.

L'invention concerne également un vecteur d'expression, caractérisé en ce qu'il comprend la cassette d'expression comprenant l'acide nucléique codant pour la protéine de fusion selon l'invention.

Par **"vecteur",** on entend un plasmide, un cosmide, un bactériophage ou un virus dans lequel est inséré un polynucléotide ou une cassette d'expression selon l'invention.
Les techniques de construction de ces vecteurs et d'insertion d'un polynucléotide de l'invention dans ces vecteurs sont connues de l'homme du métier.
De manière générale, tout vecteur capable de se maintenir, de s'autorépliquer ou de se propager dans un organisme hôte et notamment afin d'induire l'expression d'un polynucléotide ou d'un polypeptide peut être utilisé. L'homme du métier choisira les vecteurs appropriés notamment en fonction de l'organisme hôte à transformer et en fonction de la technique de transformation mise en oeuvre.
Les vecteurs de la présente invention sont notamment utilisés pour transformer un organisme hôte en vue de la réplication du vecteur et de l'expression de la protéine de fusion selon l'invention dans l'organisme hôte. De manière préférée, selon la présente invention l'organisme hôte est une cellule recombinante telle que définie ci-après.

L'invention concerne également une cellule recombinante comprenant l'acide nucléique codant pour la protéine de fusion selon l'invention, préférentiellement ledit acide nucléique est compris dans le vecteur selon l'invention.

Par **"cellule recombinante"**, on entend toute cellule contenant l'acide nucléique tel que défini ci-avant. Les cellules recombinantes selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, C127, les oeufs de Xénope, etc. Parmi les champignons, on peut citer plus particulièrement *Micro onospora, Aspergillus ssp,* ou *Trichoderma ssp.*
Comme cellules procaryotes, on préfère utiliser les bactéries suivantes : *Actinomycètes* et notamment *Streptomyces, E. coli, Bacillus,* etc... Préférentiellement, les cellules recombinantes de l'invention sont choisies dans le groupe comprenant Pichia pastoris, cellules d'insectes (cellules Sf9) CHO et les bactéries de types *E.coli.* Cette bactérie peut être cultivée en milieu riche (par exemple : milieu Lauria-Bertani) ou en milieu minimum (par exemple le milieu préparé selon la méthode décrite par Riesenberg et al 1990). Afin d'exprimer la protéine de fusion selon l'invention, l'induction est réalisée au début de la phase de croissance pendant 6 heures ou pendant toute la nuit.

Préférentiellement, la cellule recombinante selon l'invention est *E.Coli KRX.*
Les cellules recombinantes de l'invention peuvent être obtenues par toute méthode permettant d'introduire une séquence nucléotidique étrangère dans une cellule. Cette transformation peut s'effectuer par électroporation, conjugaison, fusion de protoplastes, ou toute autre technique connue de l'homme du métier.
Préférentiellement, l'acide nucléique selon l'invention est introduit dans la cellule après avoir été inséré dans un vecteur par éléctroporation, conjugaison ou toute autre technique connue de l'homme du métier.

L'invention concerne également un procédé de production d'une protéine d'intérêt, caractérisé en ce qu'il comprend les étapes suivantes :
a) obtention d'un échantillon comprenant la protéine de fusion selon l'invention ;
b) passage de l'échantillon sur une première colonne de chromatographie, recouverte de Nickel, ladite protéine de fusion se fixant à ladite colonne ;
c) élution de ladite protéine de fusion fixée à la colonne à l'étape b) ;
d) passage de l'éluat obtenu à l'étape c) sur une seconde colonne de chromatographie recouverte de streptavidine, sur laquelle est immobilisée l'entérokinase, ou la TEV protéase fusionnée à un polypeptide ou une protéine présentant une affinité pour la streptavidine, préférentiellement le steptag II, ladite protéine de fusion se fixant à ladite seconde colonne de chromatographie ;
e) clivage de ladite protéine de fusion fixée à la seconde colonne, au niveau de la séquence de clivage spécifique par l'entérokinase ;
f) récupération de la protéine d'intérêt purifiée en sortie de la deuxième colonne de chromatographie.

De manière optionnelle, le procédé selon l'invention est caractérisé en ce qu'il comprend en outre une étape b'), préalable à l'étape c), de lavage de la première colonne de chromatographie.

De manière optionnelle, le procédé selon l'invention est caractérisé en ce qu'il comprend en outre une étape d') préalable à l'étape e), d'élution de la protéine de fusion fixée à la colonne à l'étape d) et une étape d") de passage de l'éluat obtenu à l'étape d') sur une colonne de chromatographie recouverte de streptavidine, cette colonne pouvant être la même que celle de l'étape d). Dans ce cas, il faudra prévoir une étape de nettoyage de ladite colonne pour éliminer, les impuretés. Cette étape permet d'augmenter le degré de pureté de la protéine de fusion en se débarrassant des contaminants cellulaires qui ont échappés à l'étape b). Cette étape préalable à la digestion peut être intéressante pour des expériences de cristallographie de la protéine d'intérêt ou pour les protéines thérapeutiques.

La protéine récupérée à l'étape f) du procédé selon l'invention présente un degré de pureté élevé en sortie de la seconde colonne de chromatographie et les domaines de marquages de la protéine de fusion selon l'invention restent fixés à ladite seconde colonne de chromatographie.

Par **"échantillon",** on entend la cellule recombinante et/ou le milieu dans lequel elle se trouve. Le terme échantillon peut donc désigner la cellule recombinante seule, le milieu de culture seul, ou bien les deux.

La nature de l'échantillon dépend du type de culture cellulaire qui permet l'obtention de la protéine de fusion selon l'invention.

Plus spécifiquement, si la protéine de fusion se trouve dans la cellule, il est possible d'effectuer une lyse cellulaire pour la récupération de ladite protéine de fusion. Le procédé de lyse cellulaire, permettant l'obtention d'un lysat cellulaire, est bien connu de l'homme du métier.

Par **"colonne de chromatographie",** on entend une colonne permettant l'opération de chromatographie, sur lequel est fixée la phase stationnaire permettant la rétention de composé. Il est rappelé que la chromatographie est une technique d'analyse qualitative et quantitative dans laquelle un échantillon contenant une ou plusieurs espèces est entraîné par un courant de phase mobile le long d'une phase stationnaire. Chaque espèce se déplace à une vitesse propre dépendant de ses caractéristiques et de celles des deux phases, ce qui a pour conséquence une séparation des ces espèces.

Par "Cutcolumn^{®}", on entend la seconde colonne de chromatographie recouverte de streptavidine, sur laquelle est immobilisée l'entérokinase ou la TEV protéase, fusionnée à un polypeptide ou une protéine présentant une affinité pour la streptavidine, préférentiellement le steptag II.

De manière préférée, la colonne de chromatographie Cutcolum^{®} selon l'invention est caractérisée en ce que l'immobilisation de l'entérokinase ou de la TEV protéase est de type covalente.

Par **"élution"**, on entend un procédé permettant de mettre en solution un composé adsorbé sur une colonne de chromatographie.

Par **"éluant"**, on entend un liquide, permettant la séparation d'un composé adsorbé sur une colonne de chromatographie. Dans le cadre de cette invention, l'Imidazole et la Desthiobiotine sont utilisés pour éluer respectivement la colonne de nickel et la colonne de streptavidine.

De manière particulière, l'élution de la colonne recouverte de Nickel se fait à l'aide d'éluants spécifiques, tels que l'Imidazole.

De même, l'élution de la colonne recouverte de streptavidine se fait à l'aide d'éluants spécifiques, tels que... la Desthiobiotine.

Par **"éluat",** on entend le liquide récupéré en sortie de la colonne de chromatographie après remise en solution du corps adsorbé sur ladite colonne.

Par **"récupération en sortie de colonne"**, on entend l'étape du procédé selon l'invention grâce à laquelle on récupère la protéine d'intérêt qui est éluée, tandis que le domaine de marquage de la protéine de fusion selon l'invention reste accroché à la colonne. On récupère donc une protéine d'intérêt purifiée.

Préférentiellement, l'invention concerne le procédé de production d'une protéine d'intérêt, caractérisé en ce qu'il comprend en outre après l'étape a) et préalablement à l'étape b), une étape a') de quantification et/ou de suivi de la production en temps réel (par exemple par spectrophotométrie).

Plus préférentiellement, l'invention concerne le procédé de production d'une protéine d'intérêt, caractérisé en ce qu'il comprend en outre après l'étape a) et préalablement à l'étape b), une étape a'') de quantification et/ou de suivi de la production se basant sur le dosage enzymatique.

Dans le cadre de cette invention, l'étape a) peut être suivi de l'étape a') et a").

Par **"quantification"**, on entend la détermination de la concentration en protéine de fusion selon l'invention dans l'échantillon, de préférence effectuée en temps réel, c'est à dire au fur et à mesure de la production.

La quantification a pour objectif la vérification de la production en quantité suffisante, ainsi que le suivi de la production cellulaire.

La quantification en temps réel a pour avantage majeur l'abolition de l'attente de la fin de la culture cellulaire pour la vérification de l'expression et la présence de la protéine de fusion sous forme soluble dans la cellule hôte selon l'invention dans le milieu de culture.

Cette quantification peut se faire selon les techniques bien connues de l'homme du métier, comme le dosage enzymatique ou la quantification à l'aide d'un logiciel, par exemple ImageQuant TL, commercialisé par GE Healthcare.

Préférentiellement, l'invention concerne le procédé de production d'une protéine d'intérêt, caractérisé en ce que l'étape supplémentaire a') comprend les étapes suivantes : récupération des échantillons, lyse cellulaire, centrifugation, récupération du surnagent et mesure de la D.O.

Plus préférentiellement, l'invention concerne un concerne le procédé de production d'une protéine d'intérêt, caractérisé en ce que l'étape supplémentaire a") comprend les étapes suivantes :
A) mise en contact de l'échantillon avec une surface enduite de nickel, la protéine de fusion selon l'invention se fixant de manière spécifique à ladite surface ;
B) lavage afin d'éliminer les protéines non retenues
C) ajout de streptavidine, complexée à une enzyme présentant une affinité pour la streptavidine préférentiellement la peroxydase;
D) ajout d'un substrat chromogène spécifique de ladite enzyme, préférentiellement la tétraméthylbenzidine ; et
E) détection et/ou quantification de ladite protéine de fusion selon l'invention par colorimétrie, grâce à la dégradation dudit substrat chromogène.

Cette étape supplémentaire a') permet de suivre l'expression de la protéine de fusion en temps réel par l'absorbance du marqueur coloré de ladite protéine de fusion. La détection et/ou la quantification par le marqueur coloré se fait aux longueurs d'ondes d'absorption spécifique du marqueur coloré utilisé. Le cytochrome b5, qui absorbe dans des bandes du spectre de la lumière visible, est responsable d'une coloration rouge visible à l'oeil nu. La phyocyanine, quant à elle, est responsable d'une coloration bleue également visible à l'oeil nu. Les propriétés de coloration du marqueur au niveau de la protéine de fusion selon l'invention permettent de suivre et de contrôler toutes les étapes de production et de purification du procédé selon l'invention.

Par **"surface"** on entend un puits, de préférence une plaque, telle que celle utilisée en ELISA et présentant, par exemple, 96 puits. Le terme surface fait également référence à une colonne ou tout autre support susceptible d'être recouvert par une substance, capable d'être reconnue spécifiquement par une autre substance. Plus spécifiquement, par "surface enduite de nickel", on entend une surface qui a subit une étape de chélation avec les ions nickel. On citera pour exemple, Plaques Immobilizer™ - Nickel-Chelate commercialisé par la société NUNC.

Par **"substrat chromogène"**, on entend un substrat dont la dégradation par une enzyme permet l'identification d'un composé. Les tests utilisant ces substrats chromogènes peuvent être des tests enzymatiques qui reposent sur la capacité de détecter la disparition de substrat ou la formation de produit.

Certains substrats sont naturellement chromogènes. Si cela n'est pas le cas l'homme du métier dispose de nombreuses techniques permettant de lier un substrat à un chromogène.

Dans le cadre de cette invention, on pourra utiliser comme le substrat chromogène la tétraméthylbenzidine (TMB). Ce substrat est utilisé dans les techniques colorimétriques à base de peroxydase. LA TMB est stable, non-inflammable et une substance sûre par rapport aux autres chromogènes à base de peroxydase. Elle est, en outre, moins sensible à la lumière que les autres chromogènes à base de peroxydase et elle peut-être élaborée sans aucune précaution particulière. Enfin la TMB est notifiée non cancérigène selon Holland, V.R et al, *A safer substitude for benzidine in the detection of blood.*

Lors de l'oxydation, la TMB forme un produit réactionnel bleu soluble dans l'eau qui peut être mesuré par spectrophotométrie à une longueur d'onde de 650 nm. Lors de l'acidification, le produit réactionnel devient jaune avec un pic d'absorption à 450 nm.

Citons également la possibilité d'utiliser des réactions de bioluminescence, notamment à l'aide de la luciférase, isolée à partir de vers luisants. La luciférase est une enzyme permettant de catalyser la réaction de la luciférine avec l'ATP. L'oxydation de la luciférine, sous le contrôle de la luciférase, aboutit à la formation d'oxyluciférine et à l'émission de photons. Cette réaction devient alors, par exemple, un indicateur de la viabilité des cellules.

Par **"colorimétrie"** on entend une méthode d'analyse chimique ayant pour objet la détermination de la concentration d'un élément, en mesurant l'intensité de la coloration qu'il confère à l'échantillon mis en solution, ou, s'il est incolore ou peu coloré, par celle qu'il produit en présence d'un réactif spécifique donnant un composé coloré.

Le terme colorimétrie peut faire référence à une analyse quantitative permettant de déterminer la concentration d'un élément, ou une analyse qualitative permettant de détecter la présence d'un élément.

La détection par colorimétrie peut se baser sur l'utilisation d'un spectrophotomètre, c'est-à-dire un appareil qui permet de mesurer l'absorbance d'une solution homogène à une longueur d'onde donnée ou sur une région spectrale donnée. Selon la loi de Beer Lambert, l'absorbance d'une solution est proportionnelle à la concentration des substances en solution, à condition de se placer à la longueur d'onde à laquelle la substance absorbe les rayons lumineux (626 nm pour la physcocyanine et 412 nm pour le cytochrome b5).

Préférentiellement, l'invention concerne le procédé de production d'une protéine d'intérêt, caractérisé en ce qu'il comprend une étape supplémentaire g) de nettoyage de la deuxième colonne de chromatographie par élution.

Cette étape supplémentaire de nettoyage permet d'obtenir une colonne prête à être réutilisée selon le procédé de l'invention. Cette colonne peut être en outre stockée à 4°C et conservée. Cette possibilité de réutilisation de la colonne lui procure un avantage économique majeur. En effet ladite colonne présente une immobilisation covalente de l'entérokinase, (ou la TEV protéase ou bien les deux formes fusionnées pour la recherche) ce qui permettra l'amortissement économique rapide du prix onéreux de ces protéases.

Cette immobilisation permet surtout l'utilisation de protéines de fusion pour la synthèse de protéines d'intérêt avec un intérêt thérapeutique. Ces méthodes étaient jusqu'à ce jour non acceptable du fait de l'étape de digestion par une protéase qu'elles nécessitent. En effet, cette digestion pouvait provoquer la présence de trace de protéases dans le milieu comprenant la protéine d'intérêt purifiée, ce qui interdisait la commercialisation de protéine thérapeutique obtenue selon ce type de méthodes.

Préférentiellement, l'invention concerne le procédé de production d'une protéine d'intérêt, caractérisé en ce que l'obtention de l'échantillon de l'étape a) est réalisée par culture cellulaire. De manière préférée, l'échantillon est le milieu de culture et/ou le lysat cellulaire.

Par **"milieu de culture"** on entend le milieu comprenant tous les éléments nutritifs appropriés et nécessaire à la culture de la cellule recombinante selon l'invention. Pour les bactéries, ce milieu est généralement le milieu Lauria-Bertani ou un milieu synthétique préparé selon la méthode décrite par Riesenberg et al 1990) ; ce milieu étant susceptible d'être différent pour des cellules différentes.

Ainsi, par exemple, les organismes hétérotrophes ou chimiohétérotrophe ne pourront subvenir à leurs besoins qu'en présence de composés organiques. Leurs milieux de culture devront donc comporter ces éléments.

Les bactéries autotrophes quant à elles n'utilisent pour vivre et comme source d'énergie uniquement un composé minéral. Elles pourront donc se développer dans un milieu strictement minéral.

Préférentiellement, l'invention concerne le procédé de production d'une protéine d'intérêt, caractérisé en ce que la présence de la protéine de fusion selon l'invention dans l'échantillon de l'étape a) peut être contrôlée par la détection du marqueur coloré présent sur le domaine de marquage de ladite protéine de fusion.

La détection du marqueur coloré se fait aux longueurs d'ondes d'absorption spécifique du marqueur coloré utilisé. Le cytochrome b5, qui absorbe dans des bandes du spectre de la lumière visible, est responsable d'une coloration rouge visible à l'oeil nu. La phycocyanine, quant à elle, est responsable d'une coloration bleue visible à l'oeil nu ou par spectrophotométrie.

L'invention concerne également une colonne de chromatographie, recouverte de billes d'agarose-streptavidine en suspension, caractérisée en ce qu'au moins une entérokinase ou une TEV protéase est immobilisée sur ladite colonne grâce à une liaison covalente avec une protéine ayant une affinité pour la streptavidine, préférentiellement le streptag II, ledit polypeptide ou ladite protéine étant elle-même liée à la Streptavidine recouvrant la colonne.

Cette colonne de chromatographie permet la fixation et le clivage de la protéine de fusion selon l'invention.

Préférentiellement, la colonne de chromatographie selon l'invention est caractérisée en ce qu'une entérokinase est immobilisée sur ladite colonne grâce à une liaison covalente avec un polypeptide ou une protéine ayant une affinité pour la streptavidine, préférentiellement le streptag II, ladite protéine étant elle-même liée à la Streptavidine recouvrant la colonne.

Cette colonne de chromatographie peut présenter une immobilisation de l'entérokinase, pour une utilisation dans le cadre de la présente invention. Cette colonne peut également présenter l'immobilisation d'une autre enzyme ou une association d'enzymes, comme par exemple la TEV protéase ou une entérokinase fusionnée à une TEV protéase), pour une utilisation plus large en laboratoire ou en industrie.

L'invention concerne également un kit de purification d'une protéine à partir d'un échantillon comprenant la protéine de fusion selon l'invention, caractérisé en ce qu'il contient les éléments suivants :
✔ une première colonne de chromatographie recouverte de nickel ;
✔ un tampon de lavage de la dite première colonne
✔ un tampon d'élution de ladite première colonne ;
✔ une seconde colonne de chromatographie recouverte de streptavidine, sur laquelle une entérokinase est immobilisée grâce à une liaison covalente avec un polypeptide ou une protéine ayant une affinité pour la streptavidine, préférentiellement le streptag II ; et
✔ optionnellement, un second tampon d'élution de ladite seconde colonne.

De manière optionnelle, le kit pourra contenir les éléments suivants :
✔ une plaque contenant des puits, préférentiellement 96, enduits de Nickel ;
✔ la streptavidine couplée à la peroxydase,
✔ le plasmide incluant l'acide nucléique codant pour le domaine de marquage de la protéine de fusion de l'invention.

De manière optionnelle, est également immobilisée sur la seconde colonne de chromatographie au moins une autre enzyme, avantageusement la TEV protéase.

### Légende des figures

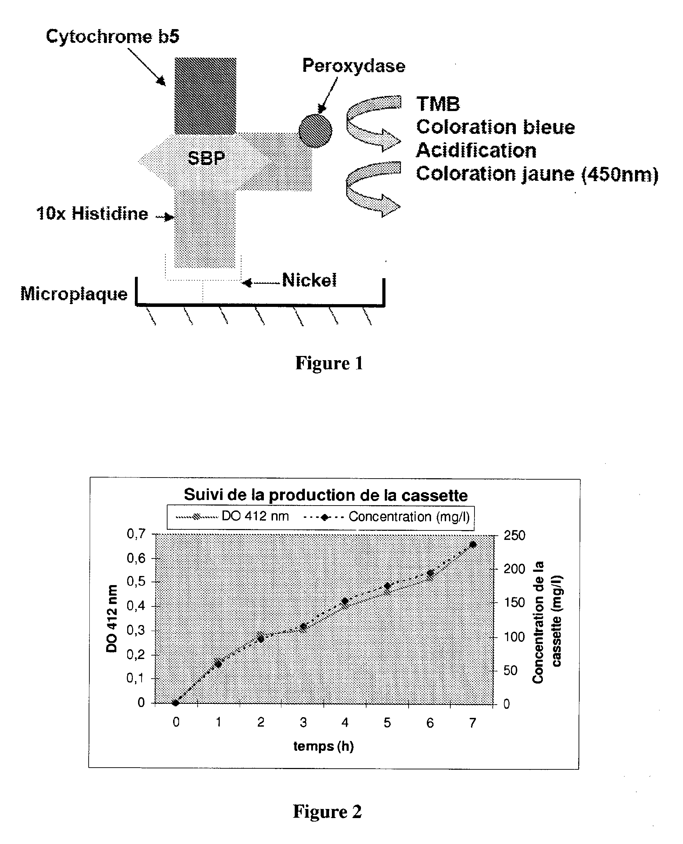

Figure 1 : Dosage enzymatique de la protéine de fusion selon l'invention. Ce schéma représente le domaine de marquage de la protéine de fusion selon l'invention (séquence de 10 histidines, SBP et cytochrome b5). Ce schéma représente également la réaction colorimétrique faisant intervenir le complexe strepatividne-peroxydase (la streptavidine se liant au niveau de la séquence SBP) et le substrat chromogène, le TMB, permettant l'apparition d'une coloration jaune.
Figure 2 : Suivi de la production de la cassette par la DO 412 nm et par la quantification enzymatique (mg/l). Cette figure présente les résultats préliminaires concernant le suivi de la production du cytochrome b5. Il représente la DO à 412 nm, caractéristique du cytochrome b5, et la concentration (dosage enzymatique) du domaine de marquage de la protéine de fusion de l'invention en mg/l, en fonction du temps en heure.

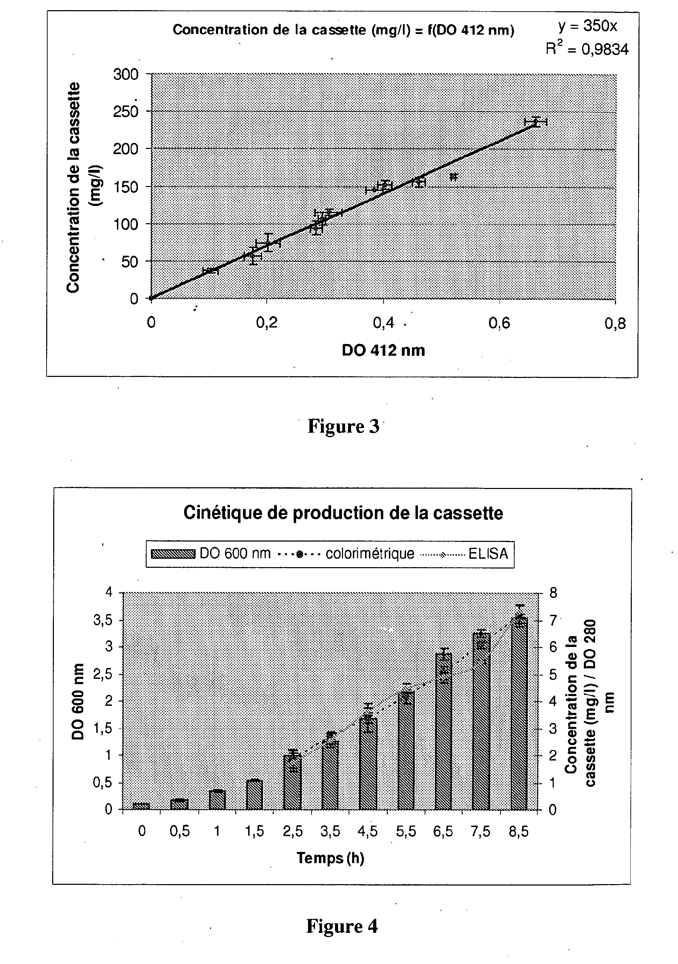

Figure 3 : Corrélation entre la DO 412 nm et la concentration de la cassette (mg/l) selon l'invention. Ce schéma représente la corrélation entre la DO à 412 nm, caractéristique du cytochrome b5, et la quantité de la cassette en mg/l.
Figure 4 : Suivi de la cinétique de production de la cassette par DO 412 nm (colorimétrique) et par dosage enzymatique (ELISA). Ce graphe représente le suivi de la biomasse (DO 600 nm) et le suivi de la production de la cassette en mg/l, rapportée à la DO 280 nm, par deux méthode : une méthode colorimétrique (DO 412 nm) sachant que 1 UDO à 412 nm correspond à 350 mg/l de la cassette et par une méthode enzymatique décrite dans la figure 1. Le suivi est réalisé pendant 7 heures après l'induction.

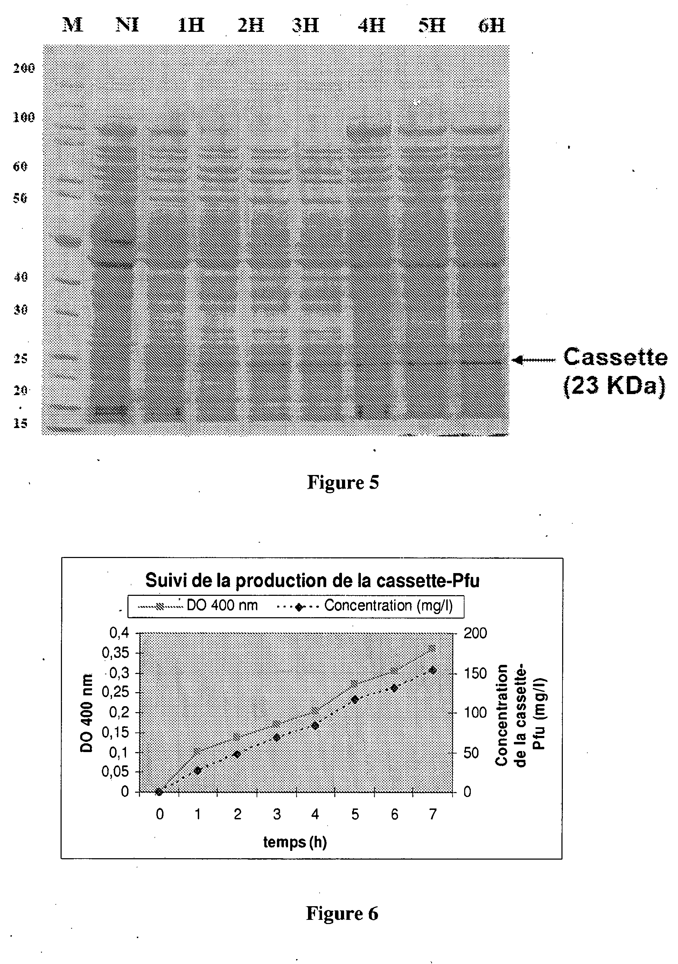

Figure 5 : Analyse par SDS-PAGE de la cinétique de production du domaine de marquage de la protéine de fusion selon l'invention. Ce gel représente l'analyse électrophorétique de la cinétique de production de la cassette étudiée dans la figure 4. Le gel montre une augmentation de l'intensité de la bande qui correspond à la cassette (23 KDa) en fonction du temps d'induction en heure.
   La piste M correspond au marqueur de poids moléculaire PageRuler. La piste NI correspond aux protéines solubles des bactéries non induites. Les pistes 1h à 7H montrent les bandes de 1 à 7 heures après l'induction.
   Dans les figures 2, 3, 4 et 5 le terme **« cassette »** désigne le domaine de marquage de la protéine de fusion selon l'invention.
Figure 6 : Suivi de la production de la cassette-Pfu par la DO 400 nm et par la quantification enzymatique (mg/l). Ce graphe présente les résultats préliminaires concernant le suivi de la production de la protéine de fusion selon l'invention. Il représente la DO à 400 nm, caractéristique de la casette-Pfu, et la concentration en protéine de fusion de l'invention en mg/l, en fonction du temps en heure.

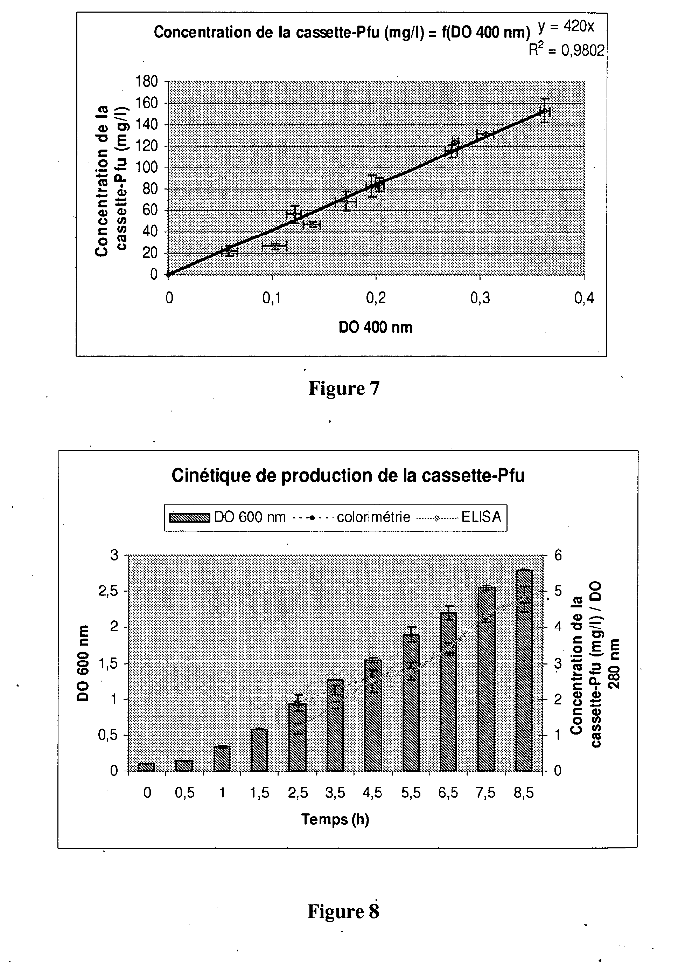

Figure 7 : Corrélation entre la DO 400 nm et la concentration de la cassette-Pfu (mg/l). Cette figure représente la corrélation entre la DO à 400 nm, caractéristique de la cassette-Pfu et la quantité de protéine de fusion en mg/l, sachant que I UDO à 400 nm correspond à 420 mg/l de la cassette-Pfu.
Figure 8 : Suivi de la cinétique de production de la cassette-Pfu par DO 400 nm (colorimétrique) et par dosage enzymatique. Cette figure représente le suivi de la biomasse (DO 600 nm) et le suivi de la production de la protéine de fusion en mg/l, rapportée à la DO 280 nm, par deux méthodes : une méthode colorimétrique (DO 400 nm) sachant que 1 UDO à 400 nm = 420 mg/l de la protéine de fusion (selon le graphique de la figure 7) et par la méthode enzymatique décrite dans la figure 1. Le suivi est réalisé pendant 7 heures après l'induction.

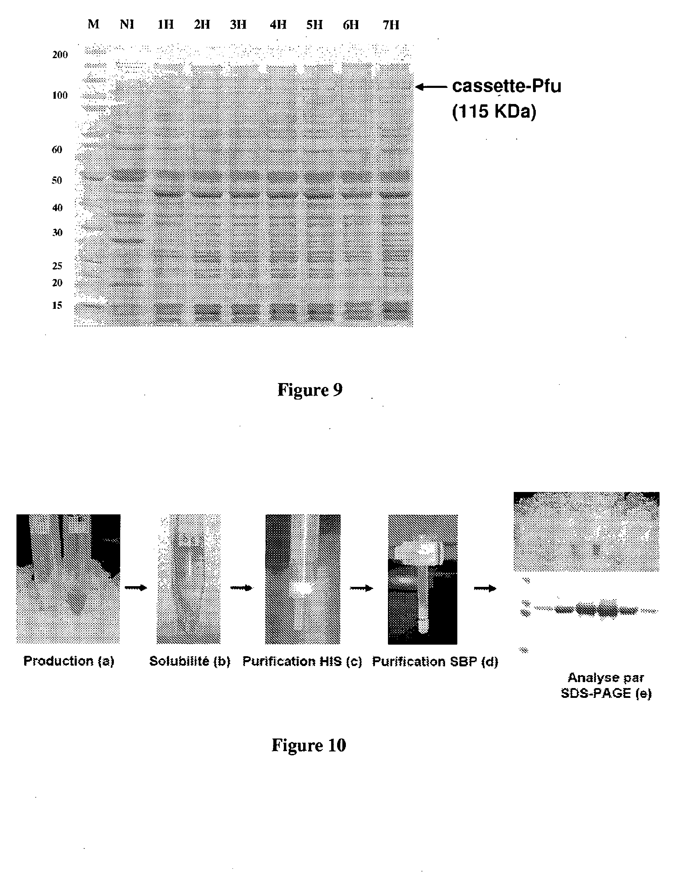

Figure 9 : Analyse par SDS-PAGE de la cinétique de production de la cassette-Pfu. Ce gel représente l'analyse électrophorétique de la cinétique de production de la cassette-Pfu étudiée dans la figure 8. Le gel montre une augmentation de l'intensité de la bande qui correspond à la cassette-Pfu (115 KDa) en fonction du temps de l'induction en heure. La piste M correspond au marqueur de poids moléculaire PageRuler. La piste NI correspond aux protéines solubles des bactéries non induites. Les pistes 1h à 7H montrent les bandes de 1 à 7 heures après l'induction.
   Dans les figures 6, 7, 8 et 9 le terme **« cassette-Pfu »** désigne un exemple de la protéine de fusion selon l'invention.
Figure 10 : Contrôle des différentes étapes du procédé de production et de purification de la protéine de fusion selon l'invention utilisant les marqueurs colorés.
Figure 11 : Domaine de marquage de la protéine de fusion selon l'invention (cassette) comprenant les éléments suivants :
   ✔ Une séquence d'histidine, constitué de 10 histidines consécutives
   ✔ La streptavidin binding protein (SBP)
   ✔ Un marqueur coloré : le cytochrome b5 ou Cyantag®
   ✔ Une séquence de clivage spécifique de l'entérokinase ou de la Tev protéase.
Figure 12 : Optimisation de l'expression de la Streptag II-Tev chez *E.coli KRX.*
   Ces deux gels montrent l'expression et la purification de la Streptag II-Tev produite chez *E.coli KRX* à deux températures d'induction différentes (30 et 25°C)
   La flèche désigne la bande correspondante à la Streptag II-Tev (27 KDa).
   La piste M correspond au marqueur de poids moléculaire PageRuler. La piste NI correspond à la fraction soluble des bactéries non induites. La piste C correspond à la fraction insoluble des bactéries induites.La piste S correspond à la fraction soluble des bactéries induites;
   La piste P correspond à la fraction purifiée.

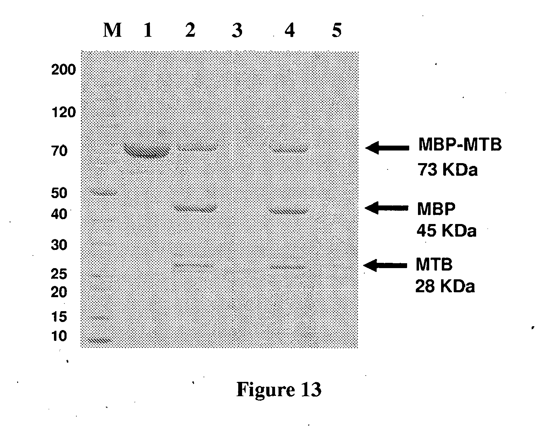

Figure 13 : Test d'activité de la Streptag II-Tev
   Ce gel SDS-PAGE montre le clivage d'une protéine de fusion (MBP-Site de clivage de la Tev-Séquence Streptag II-MTB) par la Tev-His commerciale (piste 2) et par la Tev-Streptag II produite (piste 4).
   La piste M correspond au marqueur de poids moléculaire PageRuler. La piste 1correspond à MBP-MTB non coupée. La piste 2 correspond à MBP-MTB coupée par Tev-His (invitrogen). La piste 3 correspond à Tev-His (invitrogen). La piste 4 correspond à MTB-MBP coupée par Streptag II-Tev. La piste 5 correspond à Streptag II-Tev.

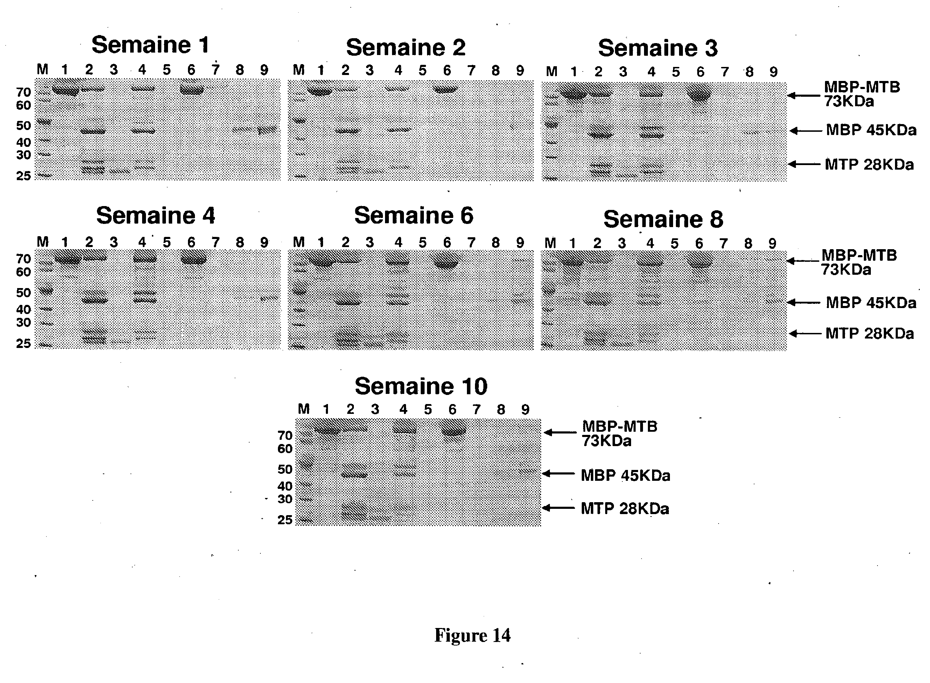

Figure 14 : Clivage d'une protéine de fusion par la Streptag II-Tev immobilisée et réutilisation de la colonne. La figure suivante montre le clivage d'une protéine de fusion (MBP-Site de clivage de la Tev-Séquence Streptag II-MTB) par la Tev-His commerciale (piste 2), par la Streptag II-Tev produite (piste 4) et par la Streptag II-Tev immobilisée sur colonne Streptavidine (pistes 7, 8 et 9). La colonne est utilisée dix fois pendant dix semaines. La piste M correspond à marqueur de poids moléculaire PageRuler. La piste 1 correspond à MBP-MTB non coupée. La piste 2 correspond à MBP-MTB coupée par Tev-His (invitrogen). La piste 3 correspond à Tev-His (Invitrogen). La piste 4 correspond à MBP-MTP coupée par Streptag II-Tev. La piste 5 correspond à Streptag II-Tev. La piste 6 correspond à MBP-MTB non coupée. La piste 7 correspond à MBP-MTB coupée par la Streptag II-Tev immobilisée (premier passage sur la colonne). La piste 8 correspond à MBP-MTB coupée par la Streptag II-Tev immobilisée (après 1 heure d'incubation de la colonne). La piste 9 correspond à MBP-MTB coupée par la Streptag II-Tev immobilisée (après 2 heures d'incubation de la colonne).

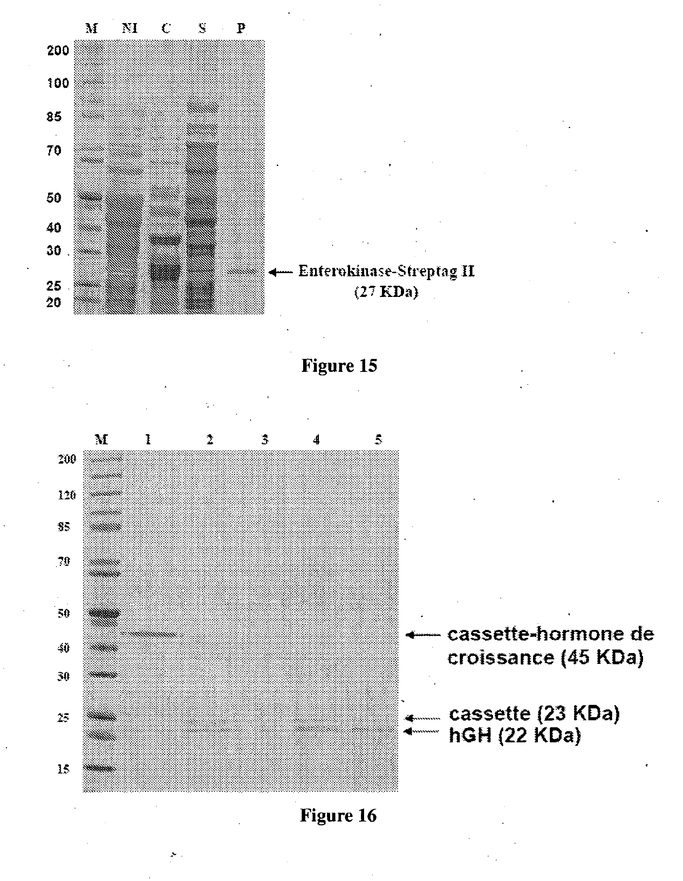

Figure 15 : Expression et purification de l'entérokinase-StreptagII chez *E.coli* KRX. La piste M correspond au marqueur de poids moléculaire PageRuler. La piste NI correspond à la fraction soluble des bactéries non induites. La piste C correspond à la fraction insoluble des bactéries induites. La piste S correspond à la fraction soluble des bactéries induites. La piste P correspond à la fraction purifiée.
Figure 16 : Clivage d'une protéine de fusion (cassette-hormone de croissance) par l'entérokinase-Streptag II libre et immobilisée. La piste M correspond au marqueur de poids moléculaire PageRuler. La piste 1 correspond à la cassette-hormone de croissance non coupée. La piste 2 correspond à la cassette-hormone de croissance coupée par l'entérokinase commerciale. La piste 3 correspond à l'entérokinase commerciale. La piste 4 correspond à la cassette-hormone de croissance coupée par l'entérokinase-Streptag II libre. La piste 5 correspond à la cassette-hormone de croissance coupée par l'entérokinase-Streptag II immobilisée.

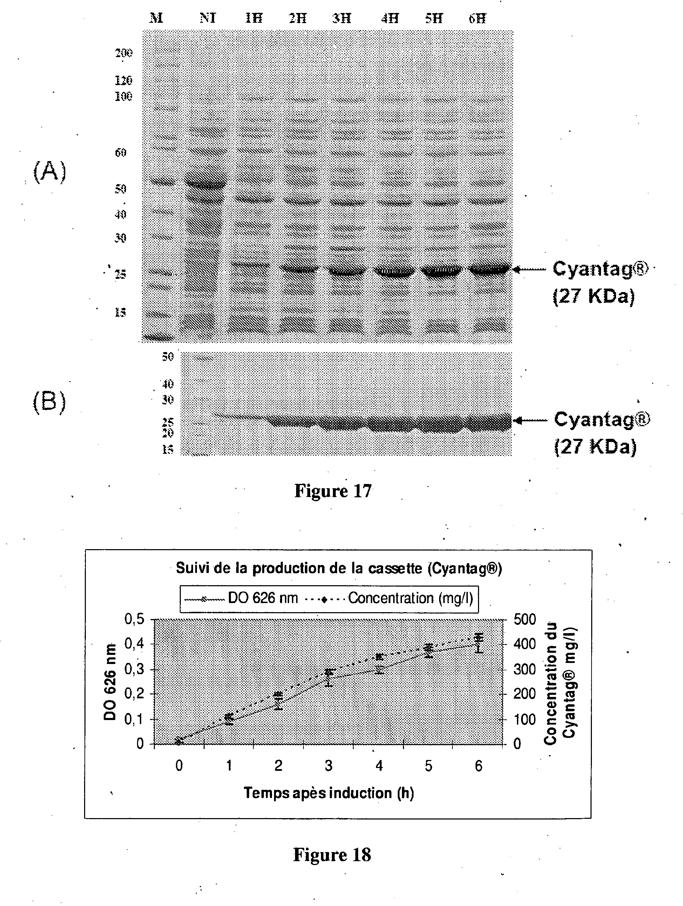

Figure 17 : Analyse par SDS-PAGE de la cinétique de production et de la purification du Cyantag®. La figure 17(A) décrit l'analyse de la cinétique de production tandis que la figure 17(B) décrit l'analyse de la double purification sur colonne de Nickel puis de streptavidine. La piste M et N correspondent respectivement au marqueur de poids moléculaire PageRuler et aux protéines solubles des bactéries non induites. Les pistes 1H à 6H correspondent respectivement à chaque heure après l'induction (fraction soluble).
Figure 18 : Suivi de la production du Cyantag® par la DO 626 nm et par dosage enzymatique et quantification densitométrique (mg/l). Cette figure représente la DO à 626 nm, caractéristique du Cyantag® , et la concentration (dosage enzymatique et quantification densitométrique) en Cyantage® en mg/l, en fonction du temps en heure.

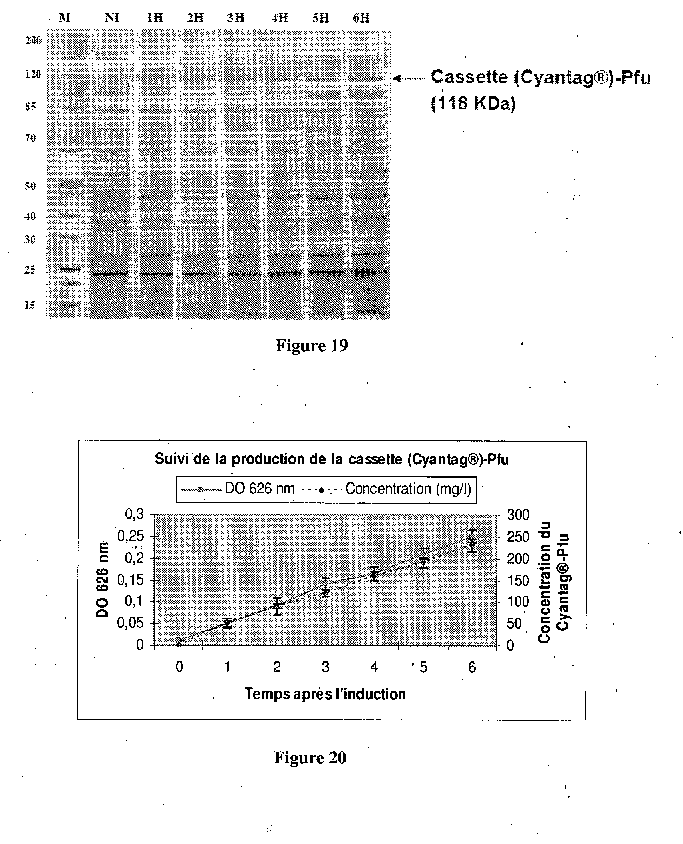

Figure 19 : Analyse par SDS-PAGE de la cinétique de production de la cassette (Cyantag®)-Pfu. La piste M et N correspondent respecivement au marqueur de poids moléculaire PageRuler et aux protéines solubles des bactéries non induites. Les pistes 1H à 6H correspondent respectivement à chaque heure après l'induction (fraction soluble).
Figure 20 : Suivi de la production de le cassette (Cyantag®)-Pfu par la DO 626 nm et par dosage enzymatique et quantification densitométrique (mg/l). Cette figure présente la DO à 626nm, caractéristique du Cyantag®, et la concentration (dosage enzymatique et quantification densitométrique) du domaine de marquage de la protéine de fusion de l'invention en mg/l, en fonction du temps en heure.

### Exemples

### Exemple 1 Quantification d'une protéine de fusion selon l'invention

Une protéine de fusion selon l'invention et comprenant le domaine de marquage [10XHis, SBP, cyto b5], un site de clivage de l'entérokinase ou de là TEV protéase et une protéine d'intérêt (étant dans cet exemple la Pfu DNA Polymérase (Pfu)) se fixe sélectivement, à l'aide des séquences d'histidines sur des plaques enduites de Nickel. Après incubation de la plaque, on elimine les protéines non retenus et on lave les puits. On ajoute au milieu la streptavidine, couplée à une enzyme, la peroxydase et on incube afin que le complexe strepavidine-peroxydase se lie sur la séquence SBP. On effectue ensuite un lavage.

On ajoute ensuite au milieu un substrat chromogène spécifique de la peroxydase, le tétraméthylbenzidine (TMB). Le TMB est oxydée par la peroxydase, ce qui a pour conséquence l'apparition d'une coloration bleue qui vire en présence d'un milieu acide vers le jaune, détectable par un spectrophotomètre. L'apparition de cette couleur témoigne de la synthèse effective de la protéine de fusion selon l'invention et de la liaison effective de la séquence d'histidine sur la surface enduite de Nickel et de la streptavidine sur la séquence SBP.

### Exemple 2 : Suivi de la production d'une protéine de fusion selon l'invention

Cet exemple concerne toujours la même protéine de fusion décrite dans l'exemple 1 (le domaine de marquage contenant le cytochrome b5 et fusionné à la Pfu).

On réalise huit cultures de la bactérie *E. coli KRX* transformée avec le vecteur recombinant pet15b-domaine de marquage-Pfu (pet15b-cassette-Pfu). Une culture va servir pour l'échantillon non induit et les autres pour les échantillons induits (toutes les heures jusqu'à la 7^{ème} heure après induction). On réalise les cultures dans 50 ml de milieu LB contenant 100 µg/ml d'Ampicilline. On cultive les bactéries recombinantes à 37°, sous une agitation de 250 rpm jusqu'à atteindre la phase exponentielle de croissance présentant une DO à 600 nm de 0,5. L'expression de protéines recombinantes est induite par 1mM d'Isopropyl-beta-thio-galactoside (IPTG) et 0,1 % Rhamnose à 37°C sous 250 rpm d'agitation. Chaque heure on retire une culture, on collecte les bactéries induites par centrifugation à 4000 rpm pendant 15 minutes et on lave le culot bactérien avec du PBS froid puis on le suspend dans un tampon de sonication (PBS contenant les inhibiteurs de protéase) et on procède à la sonication dans la glace. Enfin, on centrifuge l'extrait à 13000 rpm pendant 30 minutes à 4°C et on récupère le surnageant contenant la protéine de fusion.

On suit la production de la protéine de fusion selon l'invention par deux méthodes : En mesurant la DO à 400 nm spécifique de la présence de la protéine de fusion (cassette-Pfu) et en déterminant la quantité de cette protéine par le dosage enzymatique décrit dans la figure 1 et l'exemple 1.

### Résultats

La figure 6 présente les résultats préliminaires concernant le suivi de la production de la cassette-Pfu (n=1). Les deux courbes présentent la même allure ce qui montre une corrélation entre la DO 400 nm et la concentration de la protéine de fusion en mg/l.

Sur la base de ces résultats préliminaires et afin de trouver une relation entre la DO 400 nm et la quantité de la protéine de fusion, une culture saturée est réalisé et les protéines solubles sont extraites. A partir de cet échantillon concentré, on réalise différentes dilutions. Pour ces mêmes dilutions, on réalise la lecture de la DO 400 nm et la quantification de la protéine de fusion comme décrit dans la figure 1 et l'exemple 1. Les résultats sont présentés dans la figure 7. Le suivi de la concentration de la protéine de fusion selon l'invention en mg/l en fonction de la DO 400 nm montre une droite qui traduit une corrélation positive entre les deux paramètres. Selon le graphique, on trouve la relation suivante : la concentration de la protéine de fusion (mg/l) = 420 X DO 400 nm (1 UDO à 400 nm = 420 mg/l).

Après la détermination de cette relation, le suivi de la production de la protéine de fusion est refait selon le même protocole décrit ci-dessus mais cette fois avec n = 3. La quantification en mg/l se fait par deux méthodes : enzymatique (Figure 1) et à partir de la valeur de la DO 400 nm en se basant sur la relation ci-dessus (1 UDO à 400 nm = 420 mg/l). La figure 8 représente la cinétique de croissance bactérienne ainsi que la quantité de protéine de fusion, déterminée par les deux méthodes et rapporté par rapport à la DO 280 nm, en fonction du temps d'induction en heure.

D'après la figure, les deux méthodes de quantification donnent des résultats cohérents traduisant ainsi l'efficacité du cytochrome b5 dans le suivi de la production de la protéine de fusion selon l'invention.

On observe ainsi qu'au bout de 3 heures, la DO mesurée est de 0.171, ce qui correspond à une concentration en protéines de fusion de 71,82 mg/l (68,46 mg/l trouvé avec la méthode enzymatique) ; au bout de 4h, la DO est de 0.203, ce qui correspond à une concentration de 85,26 mg/l de protéines de fusion objet de l'invention (84,14 mg/l trouvé avec la méthode enzymatique). Enfin au bout de 7h, le spectrophotomètre mesure une DO de 0.362, ce qui correspond à une concentration d'environ 152 mg//l (153,33 mg/l méthode enzymatique).

Ces résultats montrent que le procédé de quantification selon l'invention permet ainsi le suivi de la production de la protéine de fusion selon l'invention en fonction du temps, et ceci de manière efficace et sensible.

### Exemple 3 Comparaison entre deux marqueurs dans le suivi de la production et de la purification

La production d'une protéine de fusion selon l'invention nécessite au préalable la construction de la cassette d'expression codant pour le domaine de marquage de la protéine de fusion selon l'invention.

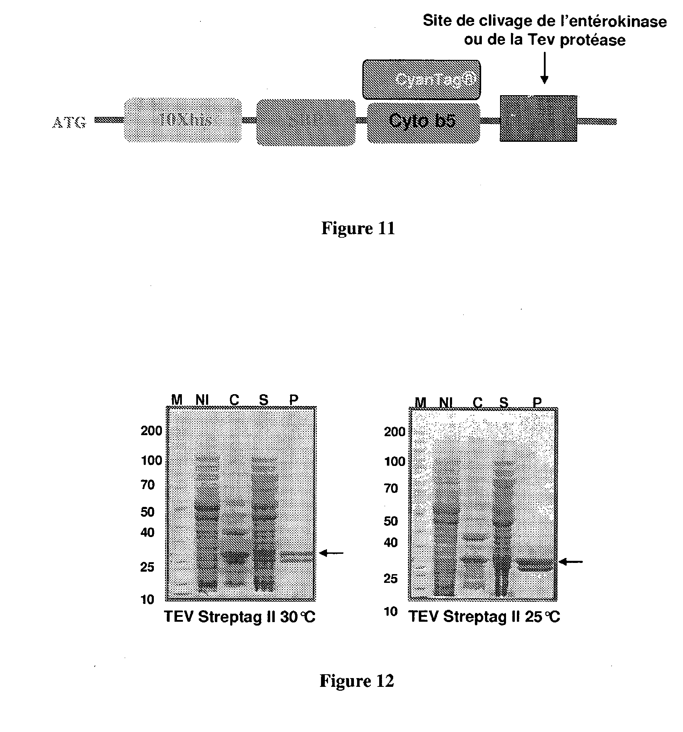

Dans cet exemple, deux cassettes d'expression différentes sont utilisées. La première cassette d'expression comprend une séquence codant pour le domaine de marquage de la protéine de fusion constitué d'une séquence histidine présentant 10 histidines, un domaine SBP, et le cytochrome b5 (figure 11). La deuxième cassette d'expression comprend une séquence codant pour le domaine de marquage de la protéine de fusion constitué d'une séquence histidine présentant 10 histidines, un domaine SBP et le domaine FMN du cytochrome p450. Ces constructions comportent également une séquence de clivage spécifique de l'entérokinase ou de la TEV protéase qui permet d'éliminer le domaine de marquage de la protéine d'intérêt.

L'acide nucléique codant pour la séquence d'histidines est réalisée par hybridation de deux oligonucléotides HIS1 et HIS2. La séquence de ces deux oligonucléotides est décrite dans le tableau 1.

On insère la séquence d'histidines dans le vecteur d'expression pET15b (Novagen). On amplifie le domaine SBP par PCR à partir du vecteur p2yX en utilisant les amorces SBP1 et SBP2 tel que décrit dans le tableau 1. On purifie le produit de PCR à partir du gel et on l'insère dans le vecteur pET15-10xHIS. On amplifie le cytochrome b5 par RT-PCR à partir des ARN totaux extrait du foie de rat.

Les techniques utilisées ici, telles que l'amplification par PCR, la purification à partir d'un gel, l'extraction des ARN totaux dont des techniques classiques bien connues de l'homme du métier.

On amplifie le cytochrome b5 par les amorces B5S et B5AS, et le cytochrome p450 par les amorces FMNS et FMNAS, ces séquences sont décrites sur le tableau 1. On réalise les réactions d'amplification PCR en utilisant la polymérase haute fidélité Pfu-Ultra (Strategene). On insère les fragments amplifiés au niveau du site du vecteur pET15b-HSBP. On séquence toutes les constructions obtenues par le protocole de séquençage ABI PRISMe 377 DNA Sequencer.

**Tableau 1 : Liste des oligonucléotides utilisés**

| Numéro de la séquence | Nom de la séquence | Séquence |
|---|---|---|
| SEQ ID No. 9 | HIS1 | |
| SEQ ID No. 10 | HIS2 | |
| SEQ ID No. 11 | SBP1 | GCGGCCGCGGCTCAGGGATGGACGAGAA |
| SEQ ID No. 12 | SBP2 | TTAATTAAGGTACCGGATCCTGGTTCAC |
| SEQ ID No. 13 | B5S | ATCGCCGAGCACTCAGACAAG |
| SEQ ID No. 14 | B5as | AAGGGTTTCCGAAGGCTT |
| SEQ ID No. 15 | FMNS | ATCAGCAGCTTTGTGGAAAAGATG |
| SEQ ID No. 16 | FMNAS | GCTGGAACTCCTCGCCAGT |

On transforme ensuite des bactéries *BL21DE3 pLysS* par les constructions pET15b-10xHis-SBP-cytob5 et pET15b-10xHis-SBP-FMN. On valide les bactéries recombinantes par PCR et digestion enzymatique. On produit les protéines dans 100 ml de milieu LB contenant 100 µg/ml d'Ampicilline et 34 µg/ml de chloramphénicol ensemencés avec des clones pET15b-His-SBp-cytob5 et pET15-His-SBP-FMN.

On cultive les bactéries recombinantes jusqu'à atteindre la phase exponentielle de croissance présentant une DO à 600 nm de 0.5 puis la production de protéines est induite par 1mM d'Isopropyl-beta-thio-galactoside (IPTG) pendant 3 heures à 37°C sous 250 rpm d'agitation. On collecte ensuite les bactéries induites par centrifugation à 4000 rpm pendant 15 minutes et on lave le culot de bactérie avec du PBS froid puis on le suspend dans un tampon de lyse contenant du PBS, 0.5 mg/ml de lysozyme, 0.5% Triton X-100, 10 mM MgCl₂, 100 µg/ml DNase I, et les inhibiteurs de protéase.

On incube ensuite la suspension bactérienne dans le tampon de lyse sous agitation à 4°C. On centrifuge l'extrait à 13000 rpm pendant 30 minutes et on récupère le surnageant contenant la protéine de fusion. On charge le surnageant sur une colonne d'affinité His-Select (Sigma). On lave la colonne avec du PBS contenant 15 mM d'Imidazole. On élue les protéines avec le tampon PBS contenant 250 mM d'imidazole. La présence du domaine de marquage selon l'invention contenant le cytochrome b5 comme marqueur coloré est vérifié à l'oeil nu (apparition de la couleur rouge). On regroupe ces fractions puis la concentration en protéines est déterminée par le protocole de Bradford. On charge alors le pool des protéines sur une colonne de streptavidine-agarose. Après le lavage au PBS, on décroche les protéines par un tampon PBS contenant 10 mM de desthiobiotine. On contrôle les fractions contenant les protéines par le chromatogramme aux longueurs d'onde d'absorption 280 nm, 412 nm pour le cytochrome b5 et 450 nm pour la FMN.

On analyse ensuite la qualité des protéines purifiées sur gel 4-12% SDS-PAGE Nupage (Invitrogen). On révèle ensuite les protéines séparées par coloration au bleu de Commassie.

### Résultats

Cet exemple décrit l'élaboration de deux cassettes présentant plusieurs domaines. La fusion de plusieurs domaines entraine parfois des encombrements stériques et l'inaccessibilité de certaines parties du domaine de marquage selon l'invention à la liaison sur les colonnes de chromatographie.

Pour tester l'accessibilité du domaine SBP, on charge les protéines purifiées de la colonne His-Select sur la colonne streptavidine agarose. La purification est suivie directement par trois longueurs d'onde : 280 nm pour la présence des protéines et 412 nm pour la présence du cytochrome b5 et 450 pour la présence du FMN. On analyse les fractions correspondantes aux maximums du pic sur GEL SDS-PAGE.Les résultats montrent que le cytochrome b5 est plus accessible comme en témoigne le rapport 412/280 proche de 1 tandis que le rapport 450/280 est proche de 0 dans le cas du domaine FMN du cytochrome p450.

Il est donc plus avantageux d'utiliser le cytochrome b5 que le domaine FMN du cytochrome p450 comme marqueur dans le cadre de cette invention.

Après induction et centrifugation des bactéries recombinantes il est facile d'observer l'expression de la protéine de fusion selon l'invention avec le cytochrome b5 (figure 10a). A cette étape, il est très difficile de savoir si la protéine est soluble ou sous forme de corps d'inclusion. Après extraction et séparation des fractions solubles et insolubles, la protéine soluble colorée est présente dans le surnageant (figure 10b). Le chargement de l'extrait de protéines permet de suivre son accrochage sur la résine d'affinité NiNTA (figure 10c). L'élution de la colonne montre que la protéine attendue est bien localisée dans les fractions colorées (figures 10d) et streptavidine (Figure 11d). L'élution de la seconde colonne de chromatographie montre que la protéine attendue est bien localisée dans les fractions colorées (Figure 11e).

Ces résultats montrent que le cytochrome b5 est d'une part un puissant traceur de l'expression de protéines recombinantes et d'autre part, il a déjà été montré qu'il permet également la solubilisation de certaines protéines normalement insolubles dans la bactérie (Mitra 2005). Sa masse moléculaire réduite moins de 15 kDa et sa coloration rend cette protéine plus avantageuse que d'autres partenaires de fusion comme les protéines GST et GFP.

### Exemple 4 Clonage et production de deux formes de l'entérokinase fusionnées au streptag II chez deux souches d'E.Coli

L'entérokinase présente un site de clivage spécifique rarement présent sur la forme native des protéines. La présente invention propose d'abolir les étapes supplémentaires de purification qu'elle engendre, en fusionnant l'entérokinase à une séquence d'affinité, le streptag II. L'immobilisation de l'entérokinase fusionnée au Streptag II sur une colonne Streptavidine permet par la suite l'élimination du domaine de marquage de la protéine de fusion et la purification de la protéine d'intérêt selon l'invention en une seule étape.

Pour ce faire, on clone et on produit deux formes de l'entérokinase fusionnées au streptag II chez deux souches de *E.coli* (*KRX et BL21pLysS*). La première forme est l'entérokinase fusionnées au streptag II du coté N terminal (Streptag II-entérokinase) et la deuxième est l'entérokinase fusionnée au streptag II du coté C terminal (entérokinase-Streptag II).

### Clonage de l'entérokinase-Streptag II :

Le gène de l'entérokinase bovine est amplifié par PCR en utilisant les deux amorces enteroC sens et enteroC antisens. Les séquences sont détaillées dans le tableau 2.

Le produit PCR est digéré par les deux enzymes de restriction NcoI et SalI et le produit de digestion est purifié à partir du gel d'agarose selon les méthodes bien connues de l'homme du métier. Le produit PCR prêt est cloné ensuite dans le vecteur pet52b préalablement digéré par NcoI et SalI et purifié à partir du gel d'agarose. Le plasmide recombinant ainsi obtenu sert à la transformation *d'E. Coli KRX* et *BL21 pLysS.*

**Tableau 2 : Séquence des amorces utilisées pour l'amplification de l'entérokinase par PCR**

| Nom de la Séquence | Séquence | Numéro de la séquence |
|---|---|---|
| enteroN sens | ATGGTACCTGATTGTCGGAGGAAGTGA | SEQ ID No. 17 |
| enteroN antisens | | SEQ ID No. 18 |
| enteroC sens | | SEQ ID No. 19 |
| enteroC antisens | | SEQ ID No. 20 |

### Production des deux formes de l'entérokinase

Les deux clones de *E.Coli KRX* transformés par les deux vecteurs recombinants pet52b-Streptag II-entérokinase et pet52b-entérokinase-StreptagII qui codent respectivement pour la Streptag II-entérokinase et l'entérokinase-StreptagII sont cultivés dans 100 ml de milieu LB-Ampicilline à 37°C, sous une agitation de 225 rpm. On cultive ces bactéries recombinantes jusqu'à atteindre la phase exponentielle de croissance à une DO de 600 nm de 0.4 à 0.6. La production est ensuite induite par 0,3 mM d'IPTG et 0.1% Rhamnose à 25°C toute la nuit.

Quant aux bactéries *E.Coli BL21 pLysS,* on les cultive dans 100 ml de milieu LB-Ampicilline-chloramphénicol à 37°C, sous une agitation de 225 rpm. On cultive ces bactéries recombinantes jusqu'à atteindre la phase exponentielle de croissance à une DO de 600 nm de 0.4 à 0.6 nm. La production est ensuite induite par 0,3 mM d'IPTG à 25°C toute la nuit.

On purifie ensuite les deux entérokinases tagués selon l'invention par chromatographie d'affinité sur une colonne de streptactin. L'élution se fait à l'aide de 10 mM de desthiobiotine.

### Résultats

Les résultats présentés sur la figure 15 indiquent que la production d'une partie de l'entérokinase- StreptagII sous forme soluble Chez *KRX* après induction avec 0.3 mM IPTG et 0.1% Rhamnose à 25°C. La même figure montre que le streptag II fusionné à l'Entérokinase en C terminal permet une bonne qualité de purification en une seule étape.

On procède, dans une seconde partie au test d'activité de l'entérokinase fusionnée au streptag II (∼27 KDa). Après purification de l'enzyme, on teste son activité sur une protéine substrat contenant le site de clivage spécifique de l'entérokinase. Dans cette exemple nous avons utilisé pour cela, par exemple, la protéine de fusion Cassette ( 10 His-SBP-Cytochromeb5)- Hormone de croissance humaine qui fait 45 KDa. D'après la figure 16, la digestion pendant 4h à 30°C montre la libération des deux partenaires de fusion : la cassette (10 His-SBP-Cytochrome b5) au niveau de 23 KDa et l'hormone de croissance humaine au niveau de 22 KDa. Le gel SDS-PAGE montre aussi une activité de l'entérokinase-streptagII comparable à celle de l'entérokinase commercialisée par INVITROGEN.

### Exemple 5 Clonage et production de la Tev protéase fusionnée au streptag II chez deux souches de E.coli

La séquence qui code pour la Tev protéase est amplifiée par PCR en utilisant les amorces Streptag II-Tev sens et Streptag II-Tev antisens. Les séquences sont détaillées dans le tableau 3.

On digère le produit PCR par NcoI et on purifie le produit de digestion à partir du gel d'agarose selon les méthodes bien connues de l'homme du métier. On clone ensuite le produit PCR dans le vecteur pet15b préalablement digéré par XhoI, traité à la klenow puis digéré par NcoI. Le plasmide recombinant ainsi obtenu sert à la transformation d'*E. Coli KRX et BL21 pLysS.*

**Tableau 3 : Séquence des amorces utilisées pour l'amplification de la Tev protéase par PCR**

| Nom de la Séquence | Séquence | Numéro de la séquence |
|---|---|---|
| Streptag II-Tev sens | | SEQ ID No. 21 |
| Tev-Streptag II-Tev antisens | AGTACACCATCATTCATGAG | SEQ ID No. 22 |

On cultive *E.Coli KRX* et *BL21 pLysS* transformées par le vecteur recombinant pet15b-Streptag II-Tev protéase dans 100 ml de milieu LB-Ampicilline (plus chloramphénicol pour *BL21 pLysS)* à 37°C, sous une agitation de 225 rpm. On cultive ces bactéries recombinantes jusqu'à atteindre la phase exponentielle de croissance à une DO de 600 nm de 0.4 à 0.6. On induit ensuite la production par 0,5 mM d'IPTG (plus 0.1% Rhamnose pour *KRX*) à 25°c toute la nuit.
On purifie ensuite la Streptag II-Tev protéase par chromatographie d'affinité sur une colonne de streptactin. L'élution se fait à l'aide de 10 mM de desthiobiotine

Les résultats présentés sur la figure 12 indiquent d'une part que l'induction s'est avérée efficace et que *E.Coli KRX* présente un niveau d'expression beaucoup plus élevé à 25°C que celui à 30°C. La même figure montre que le streptag II fusionné à la Tev protéase permet une bonne qualité de purification en une seule étape.

On procède, dans une seconde partie au test d'activité de la Tev protéase fusionnée au streptag II. Après purification de l'enzyme, on teste son activité sur une protéine substrat contenant le site de clivage spécifique de la TEV protéase. On utilise pour cela, par exemple, la protéine de fusion MBP-Site.TEV-MTB qui fait 73 KDa. D'après la figure 13, la digestion pendant 3h à 30°C montre la libération des deux partenaires de fusion : la MBP (Maltose binding protein) au niveau de 45 KDa et la MTB (nonmotor microtubule-binding protein) au niveau de 28 KDa. Le gel SDS-PAGE montre aussi une activité de la Streptag II- Tev comparable à celle de la Tev-His commercialisée par INVITROGEN.

### Exemple 6 Purification de la protéine d'intérêt (Pfu DNA polymérase)

Dans cet exemple, on produit une protéine d'intérêt, la Pfu DNA polymérase, en utilisant le procédé décrit dans la présente invention.

### a) obtention d'un échantillon comprenant la protéine de fusion selon l'invention.

Afin de produire la Pfu DNA polymérase selon l'invention, on amplifie la séquence qui code pour celle-ci par PCR en utilisant les amorces pfu sens et pfu antisens. Les séquences sont détaillées dans le tableau 4.

On digère le produit PCR par PmlI et AvrII et on purifie le produit de digestion à partir du gel d'agarose selon les méthodes bien connues de l'homme du métier. On clone ensuite le produit PCR dans le vecteur pet15b-10xHis-SBP-cytochrome b5 préalablement digéré par SwaI et AvrII. Le plasmide recombinant ainsi obtenu sert à la transformation d'*E. Coli KRX.*

**Tableau 4 : Séquences des amorces utilisées pour l'amplification de la par PCR**

| Nom de la Séquence | Séquence | Numéro de la séquence |
|---|---|---|
| Pfu sens | CACGTGGTGCCGCGCGGCAGCCATAT | SEQ ID No. 23 |
| Pfu antisens | CCTAGGCTAGGATTTTTTAATGTTA | SEQ ID No. 24 |

### -Production de la protéine de fusion

On cultive *E. coli KRX* transformée avec le vecteur recombinant petl5b-domaine de marquage-Pfu (pet15b-10xHis-SBP-cytochrome b5-Pfu) est cultivée dans 100 ml de milieu LB contenant 100 µg/ml d'Ampicilline. La culture est réalisée à 37°, sous une agitation de 250 rpm jusqu'à atteindre la phase exponentielle de croissance présentant une DO à 600 nm de 0,5. On induit la production de protéines recombinantes par 1mM d'Isopropyl-beta-thiogalactoside (IPTG) et 0,1 % Rhamnose à 37°C sous 250 rpm d'agitation. Après 6 heures d'induction, on collecte la biomasse par centrifugation à 4000 rpm pendant 15 minutes (4°C). On lave ensuite le culot bactérien avec du PBS froid et on le suspend dans 10 ml de tampon de sonication (PBS contenant les inhibiteurs de protéase) et on procède à la sonication dans la glace. Enfin, on centrifuge l'extrait à 13000 rpm pendant 30 minutes à 4°C et on récupère le surnageant contenant la protéine de fusion.

A ce stade, on peut déterminer la production de la protéine de fusion selon l'invention en se basant sur l'absorbance du cytochrome b5 (voir Exemple 2).

### b) passage de l'échantillon sur une première colonne de chromatographie (Nickel)

On passe l'échantillon précédemment obtenu sur une colonne de chromatographie recouverte de Nickel. La protéine de fusion selon l'invention se fixe sur ladite colonne. Cette fixation peut s'expliquer par la présence de la séquence d'histidine dans le domaine de marquage de la protéine de fusion selon l'invention. La colonne est lavée avec 5 fois son volume. Le tampon de lavage est le PBS contenant 15 mM Imidazole. La dynamique de l'interaction entre la protéine de fusion et la colonne est contrôlée à l' oeil nu grâce à la présence du cytochrome b5.

### c) élution de ladite protéine de fusion fixée à la colonne à l'étape b)

On élue ladite protéine de fusion fixée à la première colonne de chromatographie en passant 2 ml de tampon d'élution (PBS contenant 250 mM d'Imidazole) dans la colonne. En sortie de colonne, on collecte plusieurs fractions. On identifie les fractions contenant la protéine de fusion grâce à l'absorbance à 400 nm, caractéristique de la protéine de fusion.

### d) passage de l'éluât obtenu à l'étape c) sur une seconde colonne de chromatographie (streptavidine)

Les fractions de l'étape c) contenant la protéine de fusion sont groupées et on passe l'ensemble sur une seconde colonne de chromatographie chargée du complexe bille d'agarose-streptavidine en suspension. Sur la streptavidine est immobilisée une entérokinase fusionnée au streptag II.

La protéine de fusion selon l'invention se fixe à la seconde colonne de chromatographie. Cette fixation peut s'expliquer par la présence de la streptavidin binding protein (SBP) dans le domaine de marquage de la protéine de fusion.

### e) clivage de ladite protéine de fusion fixée

L'entérokinase, immobilisée sur la colonne de chromatographie permet de cliver la protéine de fusion au niveau de sa séquence spécifique de clivage. La colonne est incubée pendant 2 heures à 25°C.

### f) récupération de la.pfu DNA polymérase purifiée

Après deux heures d'incubation de la colonne à 25 °C, On récupère la protéine d'intérêt purifiée, débarrassée de son domaine de marquage, qui reste accroché aux billes d'agarose / streptavidine par la SBP.

### Exemple 7 Clivage d'une protéine de fusion par la Streptag II-Tev- immobilisée et réutilisation de la colonne

Cet exemple montre l'efficacité du clivage d'une protéine de fusion par la Streptag II-Tev immobilisée, ainsi que la récupération d'un des deux partenaires de fusion purifié. Ici on montre le clivage et la purification en même temps.

La protéine substrat utilisée est une protéine de fusion comportant 4 domaines : MBP-Site de clivage de la Tev-Séquence streptag II-MTB.

On immobilise la Streptag II-Tev sur une colonne remplie de billes d'agarose-streptavidine et on ajoute la protéine substrat à digérer. Le substrat se fixe sur la colonne via le tag Streptag II et rentre en contacte avec l'enzyme immobilisée. L'analyse de l'activité de la Streptag II-Tev immobilisée est effectuée par migration électrophorétique (SDS-PAGE). On analyse trois échantillons : le produit de clivage du premier passage, le produit de clivage après une heure d'incubation et après deux heures d'incubation de la colonne à 30°C. On réalise aussi deux témoins positifs : le clivage de la même protéine de fusion par la Tev-His (INVITROGEN) et par la Streptag II-Tev produite. Pour ces deux témoins, les enzymes utilisées sont sous forme libre.

La figure 13 montre un clivage de la protéine de fusion pour les deux formes libres utilisées avec libération des deux partenaires de fusion : la MBP (maltose binding protein) (45 KDa) et la StreptagII-MTB(nonmotor microtubule-binding proteins (28 KDa). Les pistes 7, 8 et 9 montrent d'une part l'activité de la Streptag II-Tev immobilisée et d'autre part la rétention du deuxième partenaire de fusion (Streptag II-MTB) sur la colonne, ceci se traduit par l'apparition d'une seule bande en sortie de colonne, qui fait 45 KDa et qui correspond à la MBP. Ainsi la protéine MBP est séparée de son partenaire de fusion et purifiée en une seule étape. La figure montre aussi la réutilisation de la colonne 10 fois pendant 10 semaines.

### Exemple 8 Clivage d'une protéine de fusion par l'entérokinase-streptagII immobilisée

Cet exemple montre l'efficacité du clivage d'une protéine de fusion par l'entérokinase-streptagII immobilisée, ainsi que la récupération d'un des deux partenaires de fusion purifié. La protéine substrat utilisée est l'hormone de croissance humaine fusionnée à la cassette.

On immobilise l'entérokinase-strepatgII sur une colonne remplie de billes d'agarose-streptavidine et on ajoute la protéine substrat à digérer. Le substrat se fixe sur la colonne via le tag Streptag II et rentre en contacte avec l'enzyme immobilisée. L'analyse de l'activité de l'entérokinase-streptagII immobilisée est effectuée par migration électrophorétique (SDS-PAGE). On analyse trois échantillons : le produit du clivage après 16h d'incubation de la colonne à 30°C. On réalise aussi deux témoins positifs : le clivage de la même protéine de fusion par l'entérokinase commerciale (INVITROGEN) et par l'entérokinase-strepatagII produite. Pour ces deux témoins, les enzymes utilisées sont sous forme libre.

La figure 16 (résultats préliminaires) montre un clivage de la protéine de fusion pour les deux formes libres utilisées avec libération des deux partenaires de fusion : la cassette (23 KDa) et l'hormone de croissance humaine (22 KDa). La pistes 5 montre d'une part l'activité de l'entérokinase-streptagII immobilisée et d'autre part la rétention du partenaire de fusion (cassette : 10 His-SBP-Cytochrome b5) sur la colonne, ceci se traduit par l'apparition d'une seule bande en sortie de colonne, qui fait 22 KDa et qui correspond à l'hormone de croissance humaine. Ainsi l'hormone de croissance humaine est séparée de son partenaire de fusion et purifiée en une seule étape.

### Exemple 9 Construction et suivi de la production de la deuxième cassette d'expression 10xHis-SBP-Cyantag®

Dans cet exemple, la nouvelle cassette d'expression comprend une séquence codant pour le domaine de marquage de la protéine de fusion constitué d'une séquence histidine présentant 10 histidines, un domaine SBP et le domaine Cyantag®. La cassette d'expression comporte également une séquence de clivage spécifique de la TEV protéase et une deuxième séquence spécifique de l'entérokinase qui permettent d'éliminer le domaine de marquage de la protéine d'intérêt.La sequence de cette protéine de fusion est SEQ ID N°25.

La séquence codante pour cette cassette d'expression est synthétisée et sous-clonée dans le vecteur pMA (Proteogenix). On digère le vecteur pMA-Cyantag® par NcoI et XhoI, on purifie le produit de digestion à partir du gel d'agarose selon les méthodes bien connues de l'homme du métier. On clone ensuite le produit de digestion (cassette d'expression) dans le vecteur pET15b préalablement digéré par les mêmes enzymes de restrictions. Le plasmide recombinant ainsi obtenu (pET15b-10xHis-SBP-Cyantag®) est utilisé pour la transformation de la souche de production : *E.Coli BL21 pLysS.*

Pour cet exemple, le marqueur utilisé au niveau du domaine de marquage de la protéine de fusion est le Cyantag® (Spirulina platensis allophycocyanin alpha subunit). L'apparition de la coloration chez la bactérie nécessite la conversion de l'hème en phycobilline et ensuite la fixation de la phycobilline au niveau du Cyantag®. Les enzymes responsables de la conversion sont : les phycocyanin alpha subunit phycocyanobilin lyase cpcE et cpcF et ceux responsables de la fixation sont l'hème oxygenase (hox1) et la phycocyanobilin ferredoxin oxidoreductase (pcyA). Les séquences nucléotidiques codant respectivement pour ces 4 enzymes sont SEQ ID N°26, 27, 28 et 29.

Les quatre séquences codantes pour cpcE, cpcF, hox1 et pcyA sont synthétisée sous formes de deux cassettes : cpcE-cpcF et hox1-pcyA (Proteogenix) les quelles sont sous-clonées séparément dans le vecteur pMK. Les deux cassettes cpcE-cpcF et hox1-pcyA sont ensuite insérées dans les deux unités transcriptionelles du vecteur pCDF Duet1. Le plasmide recombinant pCDF Duet1-cpcE-cpcF-hox1-pcyA sert à la co-transformation de *E.coli* préalablement transformée par le vecteur pET15b-Cyantag®.

Les techniques utilisées ici, telles que la transformation, la digestion enzymatique, la purification à partir d'un gel et la ligation sont classiques et bien connues de l'homme du métier.

Afin de suivre la cinétique de production de la cassette d'expression (10xHis-SBP-Cyantag®) On réalise sept cultures de la bactérie *E. coli BL21 pLysS* co-transformée avec les deux vecteurs recombinants pet15b-cassette d'expression (pet15b-10xHis-SBP-Cyantag®) et pCDF Duet1-cpcE-cpcF-hox1-pcyA. Une culture va servir pour l'échantillon non induit et les autres pour les échantillons induits (toutes les heures jusqu'à la 6^{ème} heure après induction). On réalise les cultures dans 50 ml de milieu LB contenant 100 µg/ml d'Ampicilline, 34 µg/ml de chloramphénicol et 50 µg/ml de spectinomycine. On cultive les bactéries recombinantes à 37°, sous une agitation de 250 rpm jusqu'à atteindre la phase exponentielle de croissance présentant une DO à 600 nm de 0,5. L'expression de protéines recombinantes est induite par 1mM d'Isopropyl-beta-thio-galactoside (IPTG) à 37°C sous 250 rpm d'agitation. Chaque une heure on retire une culture, on collecte les bactéries induites par centrifugation à 4000 rpm pendant 15 minutes et on lave le culot bactérien avec du PBS froid puis on le suspend dans un tampon de sonication (PBS contenant les inhibiteurs de protéase) et on procède à la sonication dans la glace. Enfin, on centrifuge l'extrait à 13000 rpm pendant 30 minutes à 4°C et on récupère le surnageant contenant la protéine de fusion. On prélève ensuite des échantillons des fractions solubles et on charge le surnageant sur une colonne d'affinité His-Select (Sigma). On lave la colonne avec du PBS contenant 15 mM d'Imidazole. On élue les protéines avec le tampon PBS contenant 250 mM d'imidazole.

On suit la production de la cassette d'expression selon l'invention par deux méthodes : En mesurant la DO à 626 nm spécifique de la présence du Cyantag® et en déterminant la quantité de ce domaine de marquage par dosage enzymatique et densitométrie à partir gel SDS-PAGE à l'aide de la caméra ImageQuant 350 et du logiciel de quantification ImageQuant TL (GE Healthcare).

### Résultats

La figure 17 présente les résultats concernant le suivi de la production de la cassette d'expression comprenant le Cyantag® comme marqueur ainsi que la qualité du domaine de marquage purifié par une double chromatographie d'affinité via les deux tags : 10xHis et SBP. L'analyse de ce gel indique l'augmentation de l'intensité de la bande (cassette : 27 KDa) en fonction du temps d'induction.

La figure 18 présente le suivi de la cinétique de production du Cyantag® par deux méthodes : la quantification par dosage enzymatique, densitométrique à partir du gel (ImageQuant TL ; GE Healthcare) et par lecture de la DO 626 nm spécifique pour le Cyantag®. Les deux courbes présentent la même allure ce qui montre une corrélation entre la DO 626 nm et la quantité de la cassette en mg/l.

### Exemple 10 : Traçage de la production d'une protéine d'intérêt par le Crantag®

Cet exemple concerne une protéine de fusion selon l'invention comprenant le domaine de marquage [10XHis, SBP, Cyantag®], un site de clivage de la TEV protéase et un deuxième site de l'entérokinase et une protéine d'intérêt (étant dans cet exemple la Pfu DNA Polymérase (Pfu)).

La production de la Pfu en fusion avec la cassette d'expression comprenant le Cyantag® comme marqueur nécessite au préalable l'insertion de la séquence codante pour la pfu dans le vecteur d'expression pet15b-10xHis-SBP-Cyantag®. Pour ceci, on récupère la séquence codante pour la Pfu par digestion enzymatique (PmlI / AvrII) à partir du plasmide pCR2.1-Pfu. On purifie le produit de digestion à partir du gel d'agarose selon les méthodes bien connues de l'homme du métier. On clone ensuite l'insert dans le vecteur petl5b-10xHis-SBP-Cyantag® préalablement digéré par SwaI et AvrII. Le plasmide recombinant ainsi obtenu sert à la transformation *d'E.Coli BL21 pLysS* contenant le vecteur pCDF Duet-cpcE-cpcF-hox1-pcyA.

On réalise sept cultures de la bactérie *E. coli BL21 pLysS* co-transformée avec les deux vecteurs recombinants pet15b-10xHis-SBP-Cyantag®-Pfu et pCDF Duet1-cpcE-cpcF-hox1-pcyA. Une culture va servir pour l'échantillon non induit et les autres pour les échantillons induits (toutes les heures jusqu'à la 6^{ème} heure après induction). On réalise les cultures dans 50 ml de milieu LB contenant 100 µg/ml d'Ampicilline, 34 µg/ml de chloramphénicol et 50 µg/ml de spectinomycine. On cultive les bactéries recombinantes à 37°, sous une agitation de 250 rpm jusqu'à atteindre une DO à 600 nm de 0,5. L'expression de protéines recombinantes est induite par 1mM d'Isopropyl-beta-thio-galactoside (IPTG) à 37°C sous 250 rpm d'agitation. Chaque une heure on retire une culture, on collecte les bactéries induites par centrifugation à 4000 rpm pendant 15 minutes et on lave le culot bactérien avec du PBS froid puis on le suspend dans un tampon de sonication (PBS contenant les inhibiteurs de protéase) et on procède à la sonication dans la glace. Enfin, on centrifuge l'extrait à 13000 rpm pendant 30 minutes à 4°C et on récupère le surnageant contenant la protéine de fusion.

On suit la production de la protéine de fusion selon l'invention par deux méthodes : En mesurant la DO à 626 nm spécifique de la présence du Cyantag® et en déterminant la quantité de la protéine de fusion par dosage enzymatique et par densitométrie à partir du gel SDS-PAGE à l'aide de la camera ImageQuant 350 et du logiciel de quantification ImageQuant TL (GE Healthcare).

### Résultats

Le suivi de la production de la Pfu DNA Polymérase fusionnée à la cassette comprenant le Cyantag® est rapporté par la figure 19. Le gel SDS-PAGE montre l'augmentation de l'expression de la protéine de fusion (118 KDa) en fonction du temps d'induction.

La figure 20 présente les résultats du suivi de la cinétique de production de la protéine de fusion par deux méthodes : la méthode de quantification de la protéine de fusion( par dosage enzymatique et par densitométrie) et la DO 626 nm. D'après la figure, les deux méthodes de quantification donnent des résultats cohérents traduisant ainsi l'efficacité du cyantag® dans le suivi de la production de la protéine de fusion selon l'invention.

## Revendications

1. Protéine de fusion **caractérisée en ce qu'**elle comprend deux domaines distincts :
✔ un domaine de marquage, comprenant les éléments suivants :
➢ une séquence d'histidines, comprenant au moins 6 histidines, préférentiellement 10 histidines,
➢ un domaine d'interaction avec la streptavidine, préférentiellement la streptavidin binding protein,
➢ un marqueur, de préférence coloré et plus préférentiellement choisi dans le groupe comprenant la Spirulina platensis allophycocyanin alpha subunit et le cytochrome b5 ainsi que leurs mutants ;
et
✔ une protéine d'intérêt,
le domaine de marquage et la protéine d'intérêt étant séparés par une séquence de clivage spécifique de l'entérokinase ou la TEV protéase.

2. Protéine de fusion selon la revendication 1, **caractérisé en ce que** la protéine d'intérêt est en position carboxy terminale.

3. Acide nucléique **caractérisé en ce qu'**il code pour la protéine de fusion selon la revendication 1 ou 2.

4. Cassette d'expression comprenant l'acide nucléique selon la revendication 3.

5. Vecteur d'expression, **caractérisé en ce qu'**il comprend la cassette d'expression selon la revendication 4.

6. Cellule recombinante comprenant l'acide nucléique selon la revendication 3, préférentiellement ledit acide nucléique est compris dans le vecteur selon la revendication 5.

7. Procédé de production d'une protéine d'intérêt, **caractérisé en ce qu'**il comprend les étapes suivantes :
a) obtention d'un échantillon comprenant la protéine de fusion selon la revendication 1 ou 2 ;
b) passage de l'échantillon sur une première colonne de chromatographie, recouverte de Nickel, ladite protéine de fusion se fixant à ladite colonne ;
c) élution de ladite protéine de fusion fixée à la colonne à l'étape b) ;
d) passage de l'éluat obtenu à l'étape c) sur une seconde colonne de chromatographie recouverte de streptavidine, sur laquelle est immobilisée l'entérokinase, ou la TEV protéase fusionnée à un polypeptide ou une protéine présentant une affinité pour la streptavidine, préférentiellement le streptag II, ladite protéine de fusion se fixant à ladite seconde colonne de chromatographie ;
e) clivage de ladite protéine de fusion fixée à la seconde colonne, au niveau de la séquence de clivage spécifique par l'entérokinase ;
f) récupération de la protéine d'intérêt purifiée en sortie de la deuxième colonne de chromatographie.

8. Procédé de production d'une protéine d'intérêt selon la revendication 7, **caractérisé en ce qu'**il comprend en outre après l'étape a) et préalablement à l'étape b), une étape a') et une étape a'') de quantification et/ou de suivi de la production respectivement par spectrophotométrie et par dosage enzymatique.

9. Procédé de production d'une protéine d'intérêt selon l'une quelconque des revendications 7 et 8, **caractérisé en ce que** l'étape supplémentaire a") comprend les étapes suivantes :
A) mise en contact de l'échantillon avec une surface enduite de nickel, la protéine de fusion se fixant de manière spécifique à ladite surface ;
B) lavage afin d'éliminer les protéines non retenues
C) ajout de streptavidine, complexée à une enzyme présentant une affinité pour la streptavidine, préférentiellement la peroxydase ;
D) ajout d'un substrat chromogène spécifique de ladite enzyme, préférentiellement la tétraméthylbenzidine ; et
E) détection et/ou quantification de ladite protéine de fusion, par colorimétrie, grâce à la dégradation dudit substrat chromogène.

10. Procédé de production d'une protéine d'intérêt selon l'une quelconque des revendications 7 à 9, **caractérisé en ce qu'**il comprend une étape supplémentaire g) de nettoyage de la deuxième colonne de chromatographie par élution.

11. Procédé de production d'une protéine d'intérêt selon l'une quelconque des revendications 7 à 10, **caractérisé en ce que** l'obtention de l'échantillon de l'étape a) est réalisée par culture cellulaire, de manière préférée, l'échantillon étant le milieu de culture et/ou le lysat cellulaire.

12. Procédé de production d'une protéine d'intérêt selon l'une quelconque des revendications 7 à 11, **caractérisé en ce que** la présence de la protéine de fusion selon la revendication 1 ou 2 dans l'échantillon de l'étape a) peut être contrôlée par la détection du marqueur coloré présent sur le domaine de marquage de ladite protéine de fusion.

13. Colonne de chromatographie permettant la fixation et le clivage de la protéine de fusion selon les revendication 1 ou 2, ladite colonne étant recouverte de streptavidine, **caractérisée en ce qu'**au moins une entérokinase, ou une TEV protéase est immobilisée sur ladite colonne grâce à une liaison covalente avec un polypeptide ou une protéine ayant une affinité pour la streptavidine, préférentiellement le streptag II, ledit polypeptide ou ladite protéine étant lié(e) à la Streptavidine recouvrant la colonne.

14. Kit de purification d'une protéine à partir d'un échantillon comprenant une protéine de fusion selon la revendication 1 ou 2, **caractérisé en ce qu'**il contient les éléments suivants :
✔ une première colonne de chromatographie recouverte de nickel ;
✔ un tampon de lavage de la dite première colonne
✔ un tampon d'élution de ladite première colonne ;
✔ une seconde colonne de chromatographie recouverte de streptavidine, sur laquelle une entérokinase est immobilisée grâce à une liaison covalente avec un polypeptide ou une protéine ayant une affinité pour la streptavidine, préférentiellement le streptag II ; et
✔ optionnellement, un second tampon d'élution de ladite seconde colonne.

## Patentansprüche

1. Fusionsprotein, **dadurch gekennzeichnet, dass** es zwei unterschiedliche Domänen umfasst:
✔ eine Markierungsdomäne, umfassend die nachfolgenden Bestandteile:
- eine Histidinsequenz, umfassend mindestens 6 Histidine, bevorzugt 10 Histidine,
- eine Domäne zur Interaktion mit dem Streptavidin, bevorzugt dem Streptavidin-bindenden-Protein,
- ein Marker, bevorzugt farbig und stärker bevorzugt ausgewählt aus der Gruppe umfassend die Spirulina-platensis-allophycocyanin-alpha-Untereinheit und das Cytochrome-b5, sowie deren Mutanten;
und
✔ ein Protein von Interesse,
wobei die Markierungsdomäne und das Protein von Interesse durch eine spezifische Spaltungssequenz der Enterokinase oder der TEV-Protease getrennt sind.

2. Fusionsprotein nach Anspruch 1, **dadurch gekennzeichnet, dass** das Protein von Interesse in einer endständigen Carboxylposition ist.

3. Nukleinsäure, **dadurch gekennzeichnet, dass** sie für das Fusionsprotein nach Anspruch 1 oder 2 kodiert.

4. Expressionskassette, umfassend die Nukleinsäure nach Anspruch 3.

5. Expressionsvektor, **dadurch gekennzeichnet, dass** er die Expressionskassette nach Anspruch 4 umfasst.

6. Rekombinante Zelle, umfassend die Nukleinsäure nach Anspruch 3, wobei die Nukleinsäure bevorzugt in dem Vektor nach Anspruch 5 umfasst ist.

7. Verfahren zur Herstellung eines Proteins von Interesse, **dadurch gekennzeichnet, dass** es die folgenden Schritte umfasst:
a) Erhalten einer Probe, die das Fusionsprotein nach Anspruch 1 oder 2 umfasst;
b) Passieren der Probe über eine erste, mit Nickel beschichtete Chromatographiesäule, wobei sich das Fusionsprotein an die Säule anlagert;
c) Eluieren des Fusionsproteins, das an die Säule von Schritt b) angelagert ist;
d) Passieren des aus Schritt c) erhaltenen Eluats über eine zweite, mit Streptavidin beschichtete Chromatographiesäule, an die die Enterokinase oder die TEV-Protease, fusioniert an ein Polypetid oder ein Protein, das Affinität für das Streptavidin aufweist, bevorzugt das Streptag II, immobilisiert ist, wobei sich das Fusionsprotein an die zweite Chromatographiesäule anlagert;
e) Spalten des Fusionsproteins, das an die zweite Säule angelagert ist, im Bereich der spezifischen Spaltungssequenz durch die Enterokinase;
f) Gewinnen des gereinigten Proteins von Interesse am Auslass der zweiten Chromatographiesäule.

8. Verfahren zur Herstellung eines Proteins von Interesse nach Anspruch 7, **dadurch gekennzeichnet, dass** es zusätzlich nach Schritt a) und vor Schritt b) einen Schritt a') und einen Schritt a") der Quantifizierung und/oder Überwachung der entsprechenden Herstellung durch Spektrophotometrie und durch enzymatisches Assay umfasst.

9. Verfahren zur Herstellung eines Proteins von Interesse nach einem der Ansprüche 7 und 8, **dadurch gekennzeichnet, dass** der zusätzliche Schritt a") die folgenden Schritte umfasst:
A) In Kontakt bringen der Probe mit einer mit Nickel beschichteten Oberfläche, wobei das Fusionsprotein auf spezifische Weise an der Oberfläche angelagert wird;
B) Waschen, um die nicht zurückgehaltenen Proteine zu entfernen;
C) Zugeben von Streptavidin, das mit einem Enzym komplexiert ist, das eine Affinität für Streptavidin aufweist, bevorzugt die Peroxidase;
D) Zugeben eines spezifischen chromogenen Substrats des Enzyms, bevorzugt das Tetramethylbenzidin; und
E) Detektion und/oder Quantifizierung des Fusionsproteins durch Kolorimetrie, mittels des Abbaus des chromogenen Substrats.

10. Verfahren zur Herstellung eines Proteins von Interesse nach einem der Ansprüche 7 bis 9, **dadurch gekennzeichnet, dass** es einen zusätzlichen Schritt g) Reinigen der zweiten Chromatographiesäule durch Eluierung umfasst.

11. Verfahren zur Herstellung eines Proteins von Interesse nach einem der Ansprüche 7 bis 11, **dadurch gekennzeichnet, dass** das Erhalten der Probe aus Schritt a) durchgeführt wird mittels Zellkultur, wobei die Probe bevorzugt Zellkulturmedium und/oder Zelllysat ist.

12. Verfahren zur Herstellung eines Proteins von Interesse nach einem der Ansprüche 7 bis 11, **dadurch gekennzeichnet, dass** die Anwesenheit des Fusionsproteins nach Anspruch 1 oder 2 in der Probe aus Schritt a) durch Detektion des farbigen Markers, der in der Markerdomäne des Fusionsproteins vorliegt, gesteuert werden kann.

13. Chromatographiesäule geeignet zur Anlagerung und Spaltung des Fusionsproteins nach Anspruch 1 oder 2, wobei die Säule mit Streptavidin beschichtet ist, **dadurch gekennzeichnet, dass** mindestens eine Enterokinase oder eine TEV-Protease an die Säule mittels einer kovalenten Bindung mit einem Polypeptide oder einem Protein, das eine Affinität für das Streptavidin aufweist, bevorzugt das Streptag II, immobilisiert ist, wobei das Polypeptid oder das Protein an Streptavidin, das die Säule beschichtet, gebunden ist.

14. Kit zur Reinigung eines Proteins aus einer Probe, umfassend ein Fusionsprotein nach Anspruch 1 oder 2, **dadurch gekennzeichnet, dass** er die folgenden Bestandteile enthält:
✔ eine erste Chromatographiesäule beschichtet mit Nickel;
✔ einen Waschpuffer der ersten Säule
✔ einen Elutionspuffer der ersten Säule;
✔ eine zweite Chromatographiesäule beschichtet mit Streptavidin, an die eine Enterokinase mittels einer kovalenten Bindung mit einem Polypeptide oder einem Protein, das eine Affinität für das Streptavidin aufweist, bevorzugt das Streptag II, immobilisiert ist;
und
✔ gegebenenfalls, einen zweiten Elutionspuffer der zweiten Säule.

## Claims

1. Fusion protein **characterized in that** it comprises two distinct domains:
✔ a labelling domain, comprising the following elements:
➢ a histidine sequence, comprising at least 6 histidines, preferentially 10 histidines,
➢ a domain for interaction with streptavidin, preferentially streptavidin binding protein,
➢ a label, which is preferably coloured and more preferentially chosen from the group comprising *Spirulina platensis* allophycocyanin alpha subunit and cytochrome b5, and also mutants thereof;
and
✔ a protein of interest,
the labelling domain and the protein of interest being separated by a cleavage sequence specific for enterokinase or TEV protease.

2. Fusion protein according to Claim 1, **characterized in that** the protein of interest is in the carboxy-terminal position.

3. Nucleic acid **characterized in that** it encodes the fusion protein according to Claim 1 or 2.

4. Expression cassette comprising the nucleic acid according to Claim 3.

5. Expression vector **characterized in that** it comprises the expression cassette according to Claim 4.

6. Recombinant cell comprising the nucleic acid according to Claim 3, said nucleic acid preferentially being included in the vector according to Claim 5.

7. Process for the production of a protein of interest, **characterized in that** it comprises the following steps:
a) obtaining a sample comprising the fusion protein according to Claim 1 or 2;
b) passing the sample over a nickel-coated first chromatography column, said fusion protein binding to said column;
c) eluting said fusion protein bound to the column in step b);
d) passing the eluate obtained in step c) over a streptavidin-coated second chromatography column, on which is immobilized enterokinase or TEV protease fused to a polypeptide or a protein which has an affinity for streptavidin, preferentially Strep-tag II, said fusion protein binding to said second chromatography column;
e) cleaving said fusion protein bound to the second column, at the level of the specific cleavage sequence, with enterokinase;
f) recovering the purified protein of interest at the outlet of the second chromatography column.

8. Method for the production of a protein of interest according to Claim 7, **characterized in that** it also comprises, after step a) and prior to step b), a step a') and a step a") of quantifying and/or monitoring the production respectively by spectrophotometry and by enzymatic assay.

9. Process for the production of a protein of interest according to either one of Claims 7 and 8, **characterized in that** the additional step a") comprises the following steps:
A) bringing the sample into contact with a nickel-coated surface, the fusion protein binding specifically to said surface;
B) washing in order to remove the non-retained proteins;
C) adding streptavidin, complexed with an enzyme which has an affinity for streptavidin, preferentially peroxidase;
D) adding a chromogenic substrate specific for said enzyme, preferentially tetramethyl-benzidine; and
E) detecting and/or quantifying said fusion protein, by colorimetry, by means of the degradation of said chromogenic substrate.

10. Process for the production of a protein of interest according to any one of Claims 7 to 9, **characterized in that** it comprises an additional step g) of cleaning the second chromatography column by elution.

11. Process for the production of a protein of interest according to any one of Claims 7 to 10, **characterized in that** the obtaining of the sample of step a) is carried out by cell culture, the sample preferably being the culture medium and/or the cell lysate.

12. Process for the production of a protein of interest according to any one of Claims 7 to 11, **characterized in that** the presence of the fusion protein according to Claim 1 or 2 in the sample of step a) can be controlled by detection of the coloured label present on the labelling domain of said fusion protein.

13. Chromatography column which allows the binding and the cleavage of the fusion protein according to Claim 1 or 2, said column being coated with streptavidin, **characterized in that** at least one enterokinase or one TEV protease is immobilized on said column by means of a covalent bond with a polypeptide or a protein which has an affinity for streptavidin, preferentially Strep-tag II, said polypeptide or said protein being bound to the streptavidin coating the column.

14. Kit for purifying a protein from a sample comprising a fusion protein according to Claim 1 or 2, **characterized in that** it contains the following elements:
✔ a nickel-coated first chromatography column;
✔ a buffer for washing said first column;
✔ a buffer for eluting said first column;
✔ a streptavidin-coated second chromatography column, on which an enterokinase is immobilized by means of a covalent bond with a polypeptide or a protein which has an affinity for streptavidin, preferentially Strep-tag II;
and
✔ optionally, a second buffer for eluting said second column.
